# EUROPÄISCHE PATENTSCHRIFT

(11) **EP 2 802 348 B1**
(45) Veröffentlichungstag und Bekanntmachung des Hinweises auf die Patenterteilung: **07.10.2020**
(21) Anmeldenummer: 13702734.8
(22) Anmeldetag: 14.01.2013
(51) Int. Cl.: A61K 39/12, A61J 1/20, A61K 39/00, A61D 1/02, A61D 7/00, A61K 39/02

(54) **KIT ZUR HERSTELLUNG EINES IMPFSTOFFS**
KIT FOR PRODUCING A VACCINE
PROCÉDÉ POUR PRODUIRE UN VACCIN

(30) Priorität: 13.01.2012 DE 102012000507; 13.01.2012 US 201261586353 P
(43) Veröffentlichungstag der Anmeldung: 19.11.2014
(73) Patentinhaber: Boehringer Ingelheim Vetmedica GmbH, 55216 Ingelheim am Rhein (DE)
(72) Erfinder: RAULEDER, Dirk Neven, 55216 Ingelheim am Rhein (DE); BEHRENS, Gerald, 55216 Ingelheim am Rhein (DE); ELBERS, Knut, 55216 Ingelheim am Rhein (DE)
(74) Vertreter: von Rohr, Hans Wilhelm
(86) Internationale Anmeldenummer: PCT/EP2013/000090
(87) Internationale Veröffentlichungsnummer: WO 2013/104550

(56) Entgegenhaltungen:
- EP-A1- 1 829 518
- WO-A2-2006/072065
- WO-A2-2009/126356
- US-A- 3 303 846
- US-A- 4 405 047
- US-A- 5 466 220
- Anonymous: "VISTA 3 VL SQ", , 1. Januar 2009 (2009-01-01), XP055062079, Gefunden im Internet: URL:http://www.merck-animal-health-usa.com /products/vista3vl5sq/overview.aspx [gefunden am 2013-05-07]
- Anonymous: "SUMMARY OF PRODUCT CHARACTERISTICS", , 1. Januar 2008 (2008-01-01), XP055062085, Gefunden im Internet: URL:http://www.ema.europa.eu/docs/en_GB/do cument_library/EPAR_-_Product_Information/ human/000832/WC500038121.pdf [gefunden am 2013-05-07]
- Anonymous: "PigProgress - Intervet/Schering-Plough Animal Health: Efficacy of simultaneous use of vaccines in swine", , 26 July 2010 (2010-07-26), XP055339547, Retrieved from the Internet: URL:http://www.pigprogress.net/Home/Genera l/2010/7/IntervetSchering-Plough-Animal-He alth-Efficacy-of-simultaneous-use-of-vacci nes-in-swine-PP004420W/ [retrieved on 2017-01-26]
- David Overbosch ET AL: "Combined Typhoid Fever and Hepatitis A Vaccine: Comparison of Immunogenicity and Safety to Concomitant Monovalent Vaccine over 3 Years", JOURNAL OF TRAVEL MEDICINE, vol. 12, no. 6, 1 November 2005 (2005-11-01), pages 319-326, XP55442156, CA ISSN: 1195-1982, DOI: 10.2310/7060.2005.12604
- "ANNEX I SUMMARY OF PRODUCT CHARACTERISTICS: Ingelvac CircoFLEX suspensions for injections for pigs; EPAR product information; European Medical Agency", , 13 February 2008 (2008-02-13), XP055165805, Retrieved from the Internet: URL:http://www.ema.europa.eu/docs/en_GB/do cument_library/EPAR_-_Product_Information/ veterinary/000126/WC500062388.pdf [retrieved on 2015-01-29]
- Anonymous: "VISTA 3 VL SQ", , 1 January 2009 (2009-01-01), XP055062079, Retrieved from the Internet: URL:http://www.merck-animal-health-usa.com /products/vista3vl5sq/overview.aspx [retrieved on 2013-05-07]
- Anonymous: "ANNEX I SUMMARY OF PRODUCT CHARACTERISTICS", , 29 November 2011 (2011-11-29), XP55637129, Retrieved from the Internet: URL:https://ec.europa.eu/health/documents/ community-register/2011/20111124113749/anx _113749_en.pdf [retrieved on 2019-10-29]

## Beschreibung

Die vorliegende Erfindung betrifft ein Kit zur Herstellung eines Impfstoffes, eine Verwendung des Kits zur Herstellung eines Impfstoffes sowie ein Verfahren zur Bereitstellung eines Impfstoffes.

Die vorliegende Erfindung betrifft insbesondere die Herstellung und Bereitstellung von Impfstoffen, auch Vakzine genannt, insbesondere im veterinärmedizinischen Bereich. Primär betrifft die vorliegende Erfindung Impfstoffe zur Immunisierung gegen die Erkrankung des Schweins mit Porcinem Circovirus, insbesondere Typ 2, auch Porcine Circovirus Disease oder PCVD genannt, und/oder mit Bakterien des Stamms Mycoplasma hyopneumoniae, auch Enzootische Pneumonie oder EP genannt.

Es ist bekannt, dass eine Vorbeugung gegen die vorgenannten Krankheiten durch Immunisierung mit Vakzinen möglich ist. Impfstoffe sind meist für die Injektion vorgesehen und müssen entsprechend steril sein. Ferner stellt jede einzelne Injektion Stress für das behandelte Lebewesen dar, weshalb die Anzahl der Impfvorgänge so gering wie möglich gehalten werden sollte.

Eine bekannte Möglichkeit zur Reduktion der Anzahl von Impfvorgängen stellen sogenannte Kombiimpfungen dar, die es ermöglichen, eine Immunisierung gegen unterschiedliche Erkrankungen in nur wenigen oder sogar nur einer einzelnen Sitzung durchzuführen. Oftmals ist eine Kombiimpfung jedoch bedingt durch Inkompatibilitäten unterschiedlicher Vakzine oder Bestandteile dieser untereinander nicht möglich. Selbst wenn eine zumindest kurzzeitige Kompatibilität erreicht werden kann, ist es notwendig, solche Kombiimpfstoffe vor Ort zu erzeugen. Insbesondere im veterinärmedizinischen Bereich müssen die Impfstoffe dann im Außenbereich zubereitet und/oder appliziert werden. Problematisch ist hier, dass es zu Verunreinigungen des Kombiimpfstoffs kommen kann, beispielsweise zu einer Kontamination mit Krankheitserregern und insbesondere wenn ein Mischgefäß mehrfach angestochen werden muss.

"Vista 3 VL 5 SQ", abgerufen unter der URL:http://www.merck-animal-health-usa.com/products/vista3vI5sq/overview.aspx betrifft ein Kit mit zwei Behältnissen, von denen ein Behältnis eine Trockensubstanz und das andere Behältnis eine Flüssigkeit zur Rehydration der Trockensubstanz aufweist. Ferner liegt eine Transfernadel zur Verbindung der Behältnisse bei.

Die US 5 466 220 A betrifft eine Misch- und Übertragungsvorrichtung, mittels derer abhängig von einer Hebelstellung Inhalte unterschiedlicher Ampullen in dieselbe Spritze aufgezogen werden können.

Die EP 1 829 518 A 1 betrifft eine Mischeinrichtung mit einem Behältnis, das ein Lösungsmittel enthält, einem weiteren Behältnis, das eine Trockensubstanz enthält und einem Verbindungsteil, das eine Doppelnadel zur Herstellung einer Verbindung zwischen den Behältnissen aufweist.

Die Anonymous: "PigProgress - Intervet/Schering-Plough Animal Health: Efficacy of simultaneous use of vaccines in swine", 26. Juli 2010, betrifft die gleichzeitige Verwendung von Schweine-Vakzinen gegen PCV2 und Mycoplasma hyopneumoniae in Schweinen.

Die WO 2009/126356 A2 betrifft die Herstellung eines Kombinationsimpfstoffes für Schweine, der sowohl ein PCV2-Antigen als auch ein Antigen für Mycoplasma Hyopneumoniae aufweist und somit zur Impfung gegen die Erkrankungen PCVD und EP verwendet werden kann.

Die JTravel Med 2005; 12: 319-326 betrifft einen Kombinationsimpfstoff zur Impfung gegen Hepatitis A und Typhus. Der Kombinationsimpfstoff ist in einer Zweikammer-Bypass-Spritze gelagert, bei der eine Kammer einen Impfstoff gegen Typhus und die andere Kammer einen Impfstoff gegen Hepatitis A enthält.

Die US 3,303,846 betrifft eine Spritze mit mehreren Kammern, innerhalb der zwei Flüssigkeiten getrennt voneinander gelagert und miteinander gemischt werden können.

Der vorliegenden Erfindung liegt die Aufgabe zu Grunde, ein Kit zur Herstellung eines Impfstoffes, eine Verwendung dieses Kit zur Herstellung eines Impfstoffes, eine Verwendung zweier Edukte zur Herstellung eines Impfstoffes sowie ein Verfahren zur Bereitstellung eines Impfstoffes anzugeben, wobei auf möglichst einfache, wenig fehleranfällige und gleichzeitig hygienische Weise auch aus nur kurzzeitig miteinander stabilen Edukten ein Impfstoff hergestellt werden kann.

Die obige Aufgabe wird gelöst durch ein Kit zur Herstellung eines Impfstoffes gemäß Anspruch 1, durch eine Verwendung eines solchen Kits zur Herstellung eines Impfstoffes gemäß Anspruch 14, oder durch ein Verfahren zur Bereitstellung eines Impfstoffes gemäß Anspruch 15. Vorteilhafte Weiterbildungen sind Gegenstand der Unteransprüche.

Ein erster Aspekt der vorliegenden Erfindung betrifft ein Kit zur Herstellung eines Impfstoffes, insbesondere zur Herstellung eines Impfstoffes zur Verwendung bei der Immunisierung gegen die Erkrankung Porcine Circovirus Disease "PCVD" und/oder Enzootische Pneumonie "EP" bzw. gegen die Infektionen mit Porcinem Circovirus, insbesondere Typ 2, und/oder Mycoplasma hyopneumoniae.

Das erfindungsgemäße Kit weist ein erstes Edukt und ein von dem ersten Edukt verschiedenes zweites Edukt auf. Ein Edukt im Sinne der vorliegenden Erfindung ist vorzugsweise jeglicher Ausgangsstoff, insbesondere ein Vakzin, ein Vakzin aufweisender Ausgangsstoff und/oder eine Komponente oder ein Ausgangsstoff für einen Impfstoff, vorzugsweise ein Antigen oder eine ein Antigen enthaltende Zusammensetzung.

Ferner weist das Kit ein erstes, mit dem ersten Edukt lediglich teilweise gefülltes Behältnis und ein zweites, das zweite Edukt aufweisendes Behältnis auf. Ein Behältnis im Sinne der vorliegenden Erfindung ist bevorzugt ein Volumen umschließendes Gebilde, vorzugsweise ausgebildet zur Aufnahme einer Flüssigkeit, insbesondere ein Gefäß, eine Flasche, ein Beutel oder dergleichen.

Das Kit weist ferner eine Adaptereinrichtung zur Herstellung einer Fluidverbindung auf, wobei die Fluidverbindung zwischen dem ersten und dem zweiten Behältnis hergestellt werden kann. Es ist also bevorzugt, dass die Adaptereinrichtung dazu ausgebildet ist, eine Fluidverbindung zwischen den Innenräumen des ersten Behältnisses und des zweiten Behältnisses herzustellen.

Ferner ist in dem vorschlagsgemäßen Kit mindestens eins der Behältnisse werkseitig mit einer Verschlusseinrichtung verschlossenen. Vorzugsweise sind beide Behältnisse werkseitig mit einer Verschlusseinrichtung verschlossenen. Es ist bevorzugt, dass ein luftdichter und/oder steriler Verschluss vorgesehen ist. Insbesondere kann es sich um eine Versiegelung, einen Gummistopfen oder dergleichen handeln, wobei die Verschlusseinrichtung bevorzugt durchbrechbar und/oder durchstoßbar ist, insbesondere reversibel. Hierdurch können die Behältnisse, also mindestens eines der Behältnisse, steril verschlossen werden.

Es ist vorgesehen, dass das zweite Behältnis mittels der Adaptereinrichtung unter erstmaligem und/oder lediglich einmaligem Durchbrechen der Verschlusseinrichtung derart mit dem ersten Behältnis verbindbar ist, dass das zweite Edukt in das erste Behältnis gelangt, insbesondere transportiert wird, und dort mit dem ersten Edukt den Impfstoff bildet. Die Verschlusseinrichtung oder Verschlusseinrichtungen sind also bevorzugt dazu ausgebildet, mittels der Adaptereinrichtung durchbrochen oder durchstoßen zu werden. Die Adaptereinrichtung ist bevorzugt derart ausgebildet, dass diese die Verschlusseinrichtung oder Verschlusseinrichtungen durchbrechen und/oder durchstoßen kann. Hierdurch kann eine Fluidverbindung zwischen dem ersten und dem zweiten Behältnis bereitgestellt werden.

Mit dem erfindungsgemäßen Kit ist es also möglich, mit lediglich zwei Behältnissen und einer Adaptereinrichtung aus zwei Edukten einen Impfstoff herzustellen, insbesondere zur Verwendung bei der Immunisierung gegen die Erkrankungen die durch Infektionen mit Porcinem Circovirus und/oder Mycoplasma hyopneumoniae verursacht werden, vorzugsweise zur Verwendung bei der Immunisierung gegen die Erkrankungen die durch Infektionen mit Porcinem Circovirus und Mycoplasma hyopneumoniae verursacht werden. Ferner wird mit dem Kit verhindert, dass andere, inkompatibele Edukte verwendet werden, insbesondere durch die Darreichung der Edukte und Behältnisse als Kit.

In vorteilhafter Weise kann der Impfstoff in dem lediglich teilweise gefüllten, ersten Behältnis gebildet werden, was verhindert, dass zusätzliche Behältnisse und/oder zusätzliche Adaptereinrichtungen verwendet werden müssen. Dies erlaubt eine möglichst einfache Anwendung. Zusätzlich wird der Materialeinsatz minimiert, da die Anzahl der benötigten Komponenten auf ein Mindestmaß reduziert wird.

Ferner ist es insbesondere vorteilhaft, dass lediglich ein einmaliges oder erstes Durchbrechen oder Durchstoßen der Verschlusseinrichtung ausreichend ist, um den Impfstoff herzustellen. Hierdurch wird nämlich vermieden, dass bei mehrfachem Durchbrechen bzw. Durchstoßen der Verschlusseinrichtung Fremdstoffe oder Krankheitserreger in den Impfstoff gelangen können.

In der vorliegenden Erfindung wird aus Gründen der Klarheit zwischen Vakzinen als mögliche Bestandteile eines oder mehrerer Edukte und dem Impfstoff als aus den Edukten hergestelltes Produkt differenziert.

Es ist also vorzugsweise so, dass mit dem Begriff "Impfstoff", auch wenn es sich hierbei ebenfalls um ein Vakzin handelt, das Endprodukt bezeichnet wird, welches vorzugsweise aus den beiden Edukten erzeugt oder hergestellt wird und/oder zur Behandlung verwendet wird. Besonders bevorzugt handelt es sich bei dem Impfstoff um einen Kombiimpfstoff, vorzugsweise enthaltend zumindest zwei von einander verschiedene Vakzine oder zumindest zwei von einander verschiedene Antigene oder zwei Antigen enthaltende Zusammensetzungen, wobei sich die die Antigen enthaltenden Zusammensetzungen zumindest in ihren Antigenen unterscheiden. Mit "Vakzinen" sind, auch wenn es sich hierbei um Impfstoffe handelt, vorzugsweise Edukte oder Bestandteile dieser gemeint, insbesondere Antigene oder Antigen enthaltenden Zusammensetzungen. Unter einem "Antigen" oder einer "Antigen enthaltende Zusammensetzung", wird vorzugsweise eine Substanz oder eine die Substanz enthaltende Zusammensetzung verstanden, die in einem Tier nach Verabreichung eine Immunantwort hervorrufen oder eine bereits bestehende Immunantwort verstärken kann. Die Differenzierung zwischen den Begriffen "Impfstoff" und "Vakzin" dient also insbesondere lediglich der Klarheit bzw. Unterscheidung des Produkts von möglichen Bestandteilen eines oder mehrerer der Edukte. Es ist folglich möglich, den Begriff "Impfstoff" durch den Begriff "Vakzin" zu ersetzen oder umgekehrt.

Ein Kit im Sinne der vorliegenden Erfindung ist insbesondere ein Verbund und/oder System mit dem ersten Behältnis, dem zweiten Behältnis und vorzugsweise der Adaptereinrichtung, die Komponenten des Kits bilden. Die Komponenten des Kits werden bevorzugt in einem Verbund in Verkehr gebracht, insbesondere in einer gemeinsamen Verpackung oder dergleichen. Es ist jedoch auch möglich, dass die genannten Komponenten einen losen Verbund zum gemeinsamen Einsatz bilden. Hierbei kann eine gemeinsame bzw. verbindende Komponente vorgesehen sein, beispielsweise eine gemeinsame Gebrauchsanleitung, Handlungsempfehlungen, Verweise in den Beschriftungen eines oder mehrerer der Komponenten des Kits oder dergleichen.

Ein weiterer, auch unabhängig realisierbarer Aspekt der vorliegenden Erfindung betrifft die Verwendung eines vorzugsweise vorschlagsgemäßen Kits zur Herstellung und/oder Bereitstellung eines Impfstoffes, insbesondere zur Immunisierung gegen die Erkrankung(en) Porcine Circovirus Disease "PCVD" und/oder Enzootische Pneumonie "EP" bzw. die Infektionen mit Porcinem Circovirus und/oder die Infektion mit Bakterien des Stammes Mycoplasma, insbesondere Mycoplasma hyopneumoniae, vorzugsweise zur Immunisierung gegen die Erkrankungen Porcine Circovirus Disease "PCVD" und/oder Enzootische Pneumonie "EP" bzw. gegen die Infektionen mit Porcinem Circovirus, insbesondere Porcinem Circovirus Typ 2 und die Infektion mit Bakterien des Stammes Mycoplasma, insbesondere Mycoplasma hyopneumoniae.

Ein weiterer, auch unabhängig realisierbarer Aspekt der vorliegenden Erfindung betrifft die Verwendung mindestens zweier Edukte zur Herstellung eines Impfstoffes, insbesondere zur insbesondere gleichzeitigen Immunisierung gegen die Erkrankung(en) Porcine Circovirus Disease "PCVD" und/oder Enzootische Pneumonie "EP", vorzugsweise zur insbesondere gleichzeitigen Immunisierung gegen die Erkrankungen Porcine Circovirus Disease "PCVD" und Enzootische Pneumonie "EP", bzw. gegen die Infektionen mit Porcinem Circovirus, insbesondere Porcinem Circovirus Typ 2 und die Infektion mit Bakterien des Stammes Mycoplasma, insbesondere Mycoplasma hyopneumoniae, wobei zur Herstellung des Impfstoffes Edukte in Behältnissen und mit einer Adaptereinrichtung eingesetzt werden. Hierbei kann ein vorschlagsgemäßes Kit eingesetzt werden.

Weiter ist vorgesehen, dass ein erstes Edukt in einem ersten, lediglich teilweise mit dem ersten Edukt gefüllten Behältnis und ein von dem ersten verschiedenes zweites Edukt in einem zweiten, das zweite Edukt aufweisenden Behältnis und eine Adaptereinrichtung ausgebildet zur Herstellung einer Fluidverbindung zwischen dem ersten und zweiten Behältnis, verwendet werden. Mindestens eines der Behältnisse ist werkseitig mit einer Verschlusseinrichtung verschlossen. Eine Fluidverbindung zwischen dem ersten und zweiten Behältnis wird mittels der Adaptereinrichtung unter erstmaligem und/oder lediglich einmaligem Durchbrechen der Verschlusseinrichtung hergestellt, um das zweite Edukt durch die Adaptereinrichtung und die Verschlusseinrichtung hindurch in das erste Behältnis zu transferieren und in dem ersten Behältnis den Impfstoff herzustellen.

Ein weiterer, auch unabhängig realisierbarer Aspekt der vorliegenden Erfindung betrifft ein Verfahren zur Bereitstellung eines Impfstoffes aus einem ersten Edukt und einem zweiten, vom ersten verschiedenen Edukt, insbesondere zur insbesondere gleichzeitigen Immunisierung gegen die Erkrankung Porcine Circovirus Disease "PCVD" und/oder Enzootische Pneumonie "EP", vorzugsweise zur insbesondere gleichzeitigen Immunisierung gegen die Erkrankungen Porcine Circovirus Disease "PCVD" und Enzootische Pneumonie "EP". Zur Herstellung des Impfstoffes kann ein vorschlagsgemäßes Kit verwendet werden.

Zwischen einem ersten, mit dem ersten Edukt lediglich teilweise gefüllten Behältnis und einem zweiten, das zweite Edukt aufweisenden Behältnis, wird durch eine Adaptereinrichtung eine Fluidverbindung derart hergestellt, dass mittels der Adaptereinrichtung unter erstmaligem und/oder lediglich einmaligem Durchbrechen und/oder Durchstechen mindestens einer werkseitig vorgesehenen Verschlusseinrichtung, die vorzugsweise mindestens eines der Behältnisse verschließt, die Fluidverbindung zwischen dem ersten und dem zweiten Behältnis hergestellt wird. Das zweite Edukt wird durch die Adaptereinrichtung und die Verschlusseinrichtung hindurch in das erste Behältnis transferiert bzw. befördert. In dem ersten Behältnis wird aus dem ersten Edukt und dem zweiten Edukt der Impfstoff gebildet.

Es ist bevorzugt, dass das erste und/oder zweite Edukt ein Vakzin, ein Antigen und/oder eine antigenenthaltende Zusammensetzung ist oder aufweist. Ferner kann sich das erste von dem zweiten Edukt unterscheiden. Das erste Edukt kann also ein erstes Vakzin, ein erstes Antigen und/oder eine ein erstes Antigen enthaltende Zusammensetzung und das zweite Edukt ein zweites, von dem ersten unterschiedliches Vakzin, ein zweites von dem ersten unterschiedliches Antigen und/oder eine Antigen enthaltende Zusammensetzung, dessen Antigen sich von dem der ersten Antigen enthaltenden Zusammensetzung unterscheidet, sein oder aufweisen. Vorzugsweise dienen das erste Vakzin und das zweite Vakzin, bzw. das erste Antigen und das zweite Antigen, bzw. die die verschiedenen Antigene enthaltenden Zusammensetzungen zur insbesondere gleichzeitigen Immunisierung gegen unterschiedliche Erkrankung oder Krankheitserreger, wie zum Beispiel gegen die Erkrankung Porcine Circovirus Disease "PCVD" und Enzootische Pneumonie "EP", bzw. zur Immunisierung gegen Porcines Circovirus, vorzugsweise Porcines Circovirus Typ 2 und gegen Mycoplasmen, vorzugsweise gegen Mycoplasma hyopneumoniae. Dadurch wird ermöglicht, einen Kombiimpfstoff zu erzeugen. Die Edukte können über Vakzine hinaus weitere Stoffe aufweisen, insbesondere Wasser, Adjuvantien und/oder Hilfsstoffe.

Es ist insbesondere bevorzugt, dass das erste Edukt lediglich eine erste der Komponenten Mycoplasma Vakzin bzw. Mycoplasma Antigen und Circovirus Vakzin bzw. Circovirus Antigen (und optional weitere Stoffe) aufweist. Das erste Edukt kann also entweder Mycoplasma Vakzin, bzw. ein oder mehrere Mycoplasma Antigene aufweisen oder alternativ Circovirus Vakzin oder ein oder mehrere Circovirus Antigene. Das erste Edukt wird bevorzugt getrennt von dem zweiten Edukt gelagert, insbesondere wenn die Edukte gemeinsam nicht langzeitstabil sind.. Das zweite Edukt weist bevorzugt lediglich die andere der Komponenten Mycoplasma Vakzin bzw. ein oder mehrere Mycoplasma Antigene und Circovirus Vakzin bzw. ein oder mehrere Circovirus Antigene auf (und optional weitere Stoffe). Wenn also das erste Edukt Mycoplasma Vakzin bzw. ein oder mehrere Maycoplasma Antigene aufweist, weist das zweite Edukt Circovirus Vakzin bzw. ein oder mehrere Circovirus Antigene auf oder umgekehrt.

Das Mycoplasma Vakzin kann attenuierte und/oder inaktivierte Bakterien, Fragmente von Bakterien oder rekombinant herstellte Teile von Mycoplasma hyopneumoniae, zumindest aber ein oder mehrere Mycoplasma hyopneumoniae Antigene aufweisen. Vorzugsweise, stammt das Mycoplasma hyopneumoniae Antigen von dem Mycoplasma hyopneumoniae J-Stamm ab bzw. handelt es sich bei dem inaktivierten Mycoplasma hyopneumoniae Bakterien um solche des J-Stamms. Weiterhin kann es sich bei dem Mycoplasma Vakzin um einen der nachfolgenden Vakzine handeln bzw. kann es sich bei dem Mycoplasma hyopneumoniae Antigen um das oder die Antigene handeln, welche in einem der nachfolgenden Vakzinen enthalten sind: Ingelvac®MycoFlex (Boehringer Ingelheim Vetmedica Inc, St Joseph, MO, USA), Porcilis M. hyo, Myco Silencer® BPM, Myco Silencer® BPME, Myco Silencer® ME, Myco Silencer® M, Myco Silencer® Once, Myco Silencer® MEH (alle von Intervet Inc., Millsboro, USA) Stellamune Mycoplasma (Pfizer Inc., New York, NY, USA), Suvaxyn Mycoplasma, Suvaxyn M. hyo, Suvaxyn MH-One (alle ehemals Fort Dodge Animal Health, Overland Park, KS, USA (jetzt Pfizer Animal Health).

Das Circovirus Vakzin kann attenuiertes und/oder inaktiviertes porcines Circovirus, vorzugsweise Typ 2, insbesondere das ORF2 Protein von Typ 2 aufweisen. Besonders bevorzugt ist die Verwendung von recombinant exprimierten ORF2 Protein des Prorcinen Circovirus Typ 2, vorzugsweise exprimiert in und gewonnen aus in vitro Zellkultur. Beispiele von ORF2 Proteinen des Procinen Circovirus Typ 2 sind unter anderem in der internationalen Patentanmeldung WO2006-072065 beschrieben. Diese haben sich als besonders vorteilhaft für eine effektive Impfung herausgestellt. Weiterhin kann es sich bei dem Circovirus Vakzin um einen der nachfolgenden Impfstoffe handeln, bzw. kann es sich bei dem Circovirus Antigen um das oder die Antigene handeln, welche in einem der nachfolgenden Vakzinen enthalten sind: Ingelvac®CircoFLEX, (Boehringer Ingelheim Vetmedica Inc, St Joseph, MO, USA), CircoVac® (Merial SAS, Lyon, France), CircoVent (Intervet Inc., Millsboro, DE, USA), or Suvaxyn PCV-2 One Dose® (Fort Dodge Animal Health, Kansas City, KA, USA).

Das Circovirus Vakzin, sofern es das ORF2 Protein enthält, beinhaltet vorzugsweise zwischen 2 µg und 150 µg, vorzugsweise zwischen 2 µg und 60 µg, weiterhin bevorzugt zwischen 2 µg und 50 µg, weiterhin bevorzugt zwischen 2µg und 40 µg, weiterhin bevorzugt zwischen 2 µg und 30 µg, weiterhin bevorzugt zwischen 2µg und 25 µg, weiterin bevorzugt zwischen 2µg und 20 µg, weiterhin bevorzugt zwischen 4 µg und 20 µg, weiterhin bevorzugt zwischen 4µg und 16 µg ORF2 Protein pro zu verabreichenden Dosis auf. Das Circovirus Vakzin ist vorzugsweise so hergestellt bzw. hergerichtet, dass 1 ml des Vakzins einer Dosis von 1 entspricht. Insbesondere kann das Circovirus Vakzin ORF2 Protein in Mengen größer als 2 µg/ml, vorzugsweise größer als 4 µg/ml und/oder weniger als 150 µg/ml, vorzugsweise weniger als 60 µg/ml, 50 µg/ml, 40 µg/ml, 30 µg/ml oder 25 µg/ml, insbesondere weniger als 20 µg/ml aufweisen. Dies ist einer zuverlässigen Applizierbarkeit zuträglich.

Das Mycoplasma Vakzin, sofern es inaktivierte Mycoplasma Bakterien, vorzugsweise inaktivierte Mycoplasma hyopneumoniae Bakterien enthält, beinhaltet vorzugsweise zwischen 10³ und 10⁹ colony forming units (CFU), vorzugsweise zwischen 10⁴ und 10⁸ (CFU), weiterhin bevorzugt zwischen 10⁵ und 10⁶ (CFU) pro zu verabreichenden Dosis, wobei die entsprechende CFU vor Inaktivierung der Bakterien eingestellt wird. Das Mycoplasma Vakzin ist vorzugsweise so hergestellt bzw. hergerichtet, dass 1 ml des Vakzins einer Dosis von 1 entspricht. Insbesondere kann das Mycoplasma Vakzin mehr als 10³ CFU/ml, vorzugsweise mehr als 10⁴ CFU/ml, insbesondere mehr als 10⁵ CFU/ml und/oder weniger als 10⁹ CFU/ml, vorzugsweise weniger 10⁸ CFU/ml, insbesondere weniger als 10⁷ CFU/ml oder 10⁶ CFU/ml inaktivierte Mycoplasma Bakterien, vorzugsweise inaktivierte Mycoplasma hyponeumoniae Bakterien, aufweisen, insbesondere vor Inaktivierung der Bakterien.

Mindestens eines der Edukte und/oder der Impfstoff kann ein Adjuvans, vorzugsweise ein polymeres Adjuvans, insbesondere Carbomer aufweisen. Vorzugsweise enthält mindestens oder genau eines der beiden Edukte, bevorzugt beide Edukte, eine Adjuvanzmenge von 500 µg bis 5 mg, vorzugsweise von 750 µg bis 2.5 mg, weiterhin bevorzugt von ungefähr 1 mg Adjuvanz pro zu verabreichenden Dosis. Die Edukte sind vorzugsweise so hergestellt bzw. hergerichtet, dass 1 ml des jeweiligen Edukts einer Dosis von 1 entspricht. Die Verwendung eines Adjuvanz, vorzugsweise eine polymeren Adjuvanz, wie beispielsweise Carbomer hat sich in Bezug auf die Effizienz der Immunisierung bzw. die Wirkdauer als vorteilhaft erwiesen. Der Einsatz alternativer und/oder zusätzlicher Adjuvantien ist jedoch nicht ausgeschlossen.

Es hat sich ferner als vorteilhaft erwiesen, wenn das Gesamtvolumen des ersten Behältnisses das Volumen des ersten Edukts mindestens um das Volumen des zweiten Edukts übersteigt, insbesondere um mehr als 2 %, vorzugsweise mehr als 5 %, insbesondere mehr als 8 %. Hierdurch kann sichergestellt werden, dass in dem ersten Behältnis ein ausreichendes Volumen für das zweite Edukt zur Verfügung steht. Zudem wird durch das Volumen des ersten Behältnisses, dasüber die Summe der Volumina des ersten und zweiten Edukts hinausgeht, eine effektive Vermischung der Edukte sichergestellt, insbesondere wenn das erste Behältnis nach dem Transfer des zweiten Edukts in das erste Behältnis bewegt wird.

Es ist also einerseits bevorzugt, dass das Gesamtvolumen des ersten Behältnisses die Summe der Volumina der Edukte überschreitet. Andererseits wird das erste Behältnis während und/oder nach dem Transfer des zweiten Edukts in das erste Behältnis bewegt, um ein homogene Vermischung und/oder Reaktion zu erreichen oder zu beschleunigen.

Gemäß eines weiteren Aspekts der vorliegenden Erfindung kann das erste Behältnis zum Einsatz in oder mit einer Injektionseinrichtung verwendet werden. Insbesondere handelt es sich um eine mehrfach verwendbare Injektionseinrichtung, beispielsweise eine Injektionspistole, ein Druckinjektor und/oder eine Selbstfüllerspritze, wie sie beispielsweise bei Impfungen größerer Tierbestände zum Einsatz kommen. Hierdurch kann nochmals die Anzahl der Durchbrechungen der Verschlusseinrichtung des ersten Behältnisses minimiert werden. Insbesondere kann die Anzahl der Durchbrechungen auf zwei reduziert werden, und zwar eine Durchbrechung für die Zubereitung und eine zweite Durchbrechung für die Entnahme des Impfstoffes. In besonders vorteilhafter Weise kann die Adaptereinrichtung zur Verbindung mit der Injektionseinrichtung verwendet werden, womit die Anzahl der Durchbrechungen auf eins reduziert werden kann.

Das erfindungsgemäße Kit kann eine Injektionseinrichtung aufweisen oder einer solchen zugeordnet sein. Insbesondere kann eine Öffnung, ein Flansch oder ein sonstiges Anschluss- oder Abschlusselement des ersten Behältnisses und/oder der Adaptereinrichtung derart ausgebildet sein, dass ein unmittelbarer Einsatz in einer oder mit einer Injektionseinrichtung ermöglicht wird. Ferner kann die Öffnung, der Flansch oder das sonstige Anschluss- und/oder Abschlusselement spezifisch für den Anschluss an eine bestimmte Injektionseinrichtung ausgebildet sein. Hierdurch kann abermals die Wahrscheinlichkeit einer fehlerhaften Anwendung, insbesondere falscher Wirkstoffmengen oder Applikationsmethoden, verringert werden.

Eines oder mehrere der Behältnisse können neben dem jeweiligen Edukt ein Gas, insbesondere Schutzgas aufweisen, wodurch die Langzeitstabilität des jeweiligen Edukts verbessert werden kann. Ferner kann die Adaptereinrichtung dazu ausgebildet sein, das Gas aus dem ersten Behältnis in das zweite Behältnis zu überführen, wenn das zweite Edukt aus dem zweiten Behältnis in das erste Behältnis transferiert wird, insbesondere unter Ausschluss der umgebenden Atmosphäre. Hierdurch kann sichergestellt werden, dass eine Beeinflussung eines oder mehrerer der Edukte und/oder des Impfstoffes, insbesondere durch Oxidation oder dergleichen, ausgeschlossen ist und/oder ein Druckausgleich ermöglicht wird.

Weitere Aspekte, Einzelheiten, Merkmale, Eigenschaften und Vorteile der vorliegenden Erfindung ergeben sich aus dem Patentansprüchen und aus der zeichnerischen Darstellung und nachfolgenden Beschreibung einer bevorzugten Ausführungsform des vorschlagsgemäßen Kits zur Herstellung eines Impfstoffes. Es zeigt:
- Fig. 1: ein erstes Behältnis, teilweise gefüllt mit einem ersten Edukt;
- Fig. 2: ein zweites Behältnis mit einem zweiten Edukt;
- Fig. 3: eine Adaptereinrichtung zur fluidischen Verbindung der Behältnisse;
- Fig. 4: ein zweites Behältnis mit eingesetzter Adaptereinrichtung;
- Fig. 5: ein erstes und ein zweites Behältnis, die über eine Adaptereinrichtung verbunden sind;
- Fig. 6: ein erstes Behältnis mit Impfstoff;
- Fig. 7: eine Injektionseinrichtung mit angesetzten ersten Behältnis.

In den Figuren werden für gleiche oder ähnliche Teile dieselben Bezugszeichen verwendet, wobei entsprechende oder vergleichbare Eigenschaften und Vorteile erreicht werden können, auch wenn von einer wiederholten Beschreibung abgesehen worden ist.

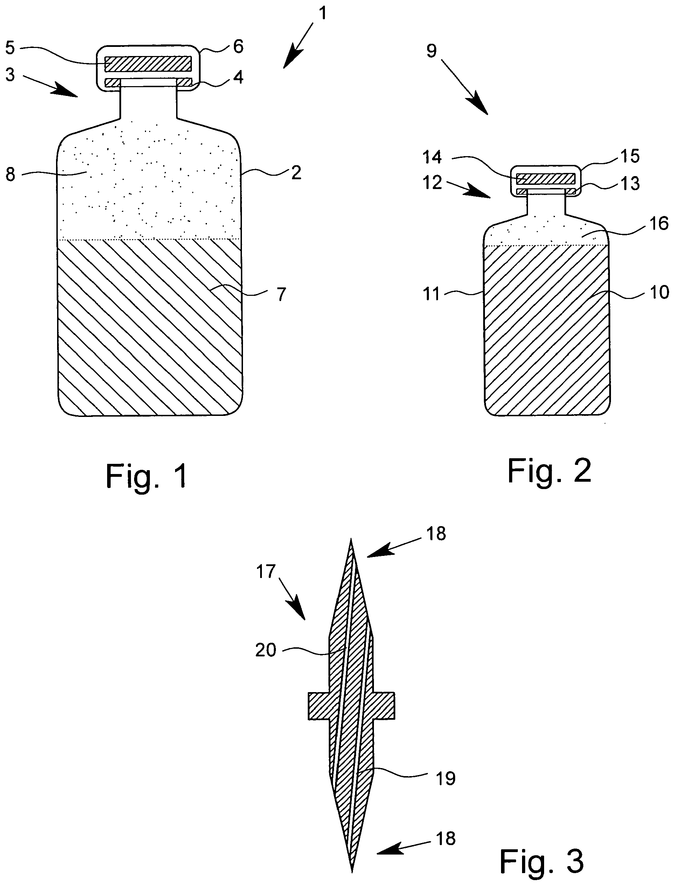

Fig. 1 zeigt ein erstes Behältnis 1 mit einer ein Volumen begrenzenden Wandung 2. Das Behältnis 1 weist einen Öffnungsbereich 3 auf, der einen Flansch 4 aufweisen kann, beispielsweise eine Ringschulter. Bevorzugt ist das Behältnis 1 durch eine Verschlusseinrichtung 5 verschlossenen, insbesondere steril und/oder luftdicht. Hierzu kann es vorgesehen sein, dass die Verschlusseinrichtung 5 fest an dem Flansch 4 anliegt, insbesondere an diesen angedrückt wird.

Beispielsweise kann ein Klemmelement 6, insbesondere ein Klemmring, vorgesehen sein, der die Verschlusseinrichtung 5 gegen den Flansch 4 presst. Das Klemmelement 6 kann metallisch sein und insbesondere Aluminium oder Edelstahl aufweisen. Alternativ oder zusätzlich ist es möglich, dass das Klemmelement 6 Kunststoff aufweist oder aus Kunststoff besteht. Das Klemmelement 6 ist bevorzugt derart ausgebildet, dass es die Verschlusseinrichtung 5 gegen den Flansch 4 drückt und hierdurch eine Abdichtung des ersten Behältnisses 1, insbesondere gegen die Umgebung und/oder Atmosphäre, ermöglicht.

Im Darstellungsbeispiel verschließt bzw. versiegelt die Verschlusseinrichtung 5 das erste Behältnis 1. Die Verschlusseinrichtung 5 ist vorzugsweise durchbrechbar bzw. durchstoßbar, beispielsweise mit einem Dorn, einer Nadel, einer Hohlnadel, einem Doppeldorn oder dergleichen. Besonders bevorzugt ist die Verschlusseinrichtung 5 dermaßen durchbrechbar bzw. durchstoßbar ausgebildet, dass ein reversibler Verschluss erreicht wird, die Verschlusseinrichtung 5 also insbesondere auch nach Durchstoßen bzw. Durchbrechen und Entfernen des entsprechenden Mittels wieder einen insbesondere luftdichten und/oder sterilen Verschluss ermöglicht. Die Verschlusseinrichtung 5 kann Gummi, insbesondere Halobutyl-Typl-Gummi aufweisen und/oder als Gummistopfen ausgebildet sein. Die Verschlusseinrichtung 5 kann aber auch aus anderen Materialien, insbesondere aus Materialien, die auch für die Wandung 2 eingesetzt werden, bestehen oder solche Materialien aufweisen.

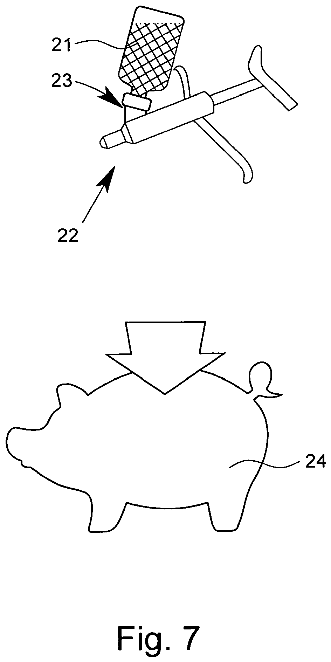

Die Wandung 2 des ersten Behältnisses 1 weist vorzugsweise einen sterilisierbaren Werkstoff auf, vorzugsweise Glas, Polyethylen, Polyethylen hoher Dichte (HDPE), Ethylenvinylacetat (EVA), Halobutyl-Typl-Gummi und/oder silikonisiertes Chlorbutyl. Die Wandung 2 kann starr oder flexibel ausgebildet sein. Das erste Behältnis 1 kann insbesondere als Flasche, Beutel, Dose oder dergleichen ausgebildet sein. Insbesondere kann das erste Behältnis 1 eine Kartusche, eine Patrone, ein Einsatz oder eine Ein- oder Ansatzeinrichtung für eine Injektionsvorrichtung 22 sein (vergleiche Figur 7), worauf später noch näher eingegangen wird.

Im Darstellungsbeispiel aus Figur 1 ist das Behältnis 1 lediglich teilweise mit einem ersten Edukt 7 gefüllt. Das Behältnis 1 ist beispielsweise mit dem ersten Edukt zu weniger als 70 %, vorzugsweise weniger als 50 %, insbesondere weniger als 45 % und/oder zu mehr als 10 %, vorzugsweise mehr als 20 %, insbesondere mehr als 30 % gefüllt.

Neben dem ersten Edukt kann das erste Behältnis 1 ein Gas 8 aufweisen, insbesondere ein Schutzgas, Edelgas, inertes Gas oder dergleichen. Hierdurch kann die Haltbarkeit des ersten Edukts 7 verbessert werden.

Vorzugsweise ergibt die Summe der Volumina des ersten Edukts 7 und des Gases 8 das Gesamtvolumen des ersten Behältnisses 1, vorzugsweise also das von der Wandung 2 des ersten Behältnisses 1, insbesondere mit der Verschlusseinrichtung 5, umschlossene Volumen, oder ein geringeres Volumen.

Figur 2 zeigt ein zweites Behältnis 9 mit einem zweiten Edukt 10. Das zweite Behältnis 9 kann eine Wandung 11, einen Öffnungsbereich 12, einen Flansch 13, eine Verschlusseinrichtung 14, und/oder ein Klemmelement 15 aufweisen, welche vorzugsweise gleiche oder ähnliche Eigenschaften wie die entsprechenden Elemente des ersten Behältnisses 1 aufweisen können, weshalb an dieser Stelle von einer wiederholten Beschreibung abgesehen wird. Daher wird im Folgenden lediglich auf mögliche Unterschiede des zweiten Behältnisses 9 zum ersten Behältnis 1 Bezug genommen.

Das zweite Behältnis 11 ist bevorzugt mit einer zumindest teilweise flexiblen Wandung 11 ausgebildet, insbesondere wobei durch Eindrücken der Wandung 11 Druck auf das zweite Edukt 10 ausgeübt werden kann. Dies kann den Austrag bzw. Transfer des zweiten Edukts 10 begünstigen.

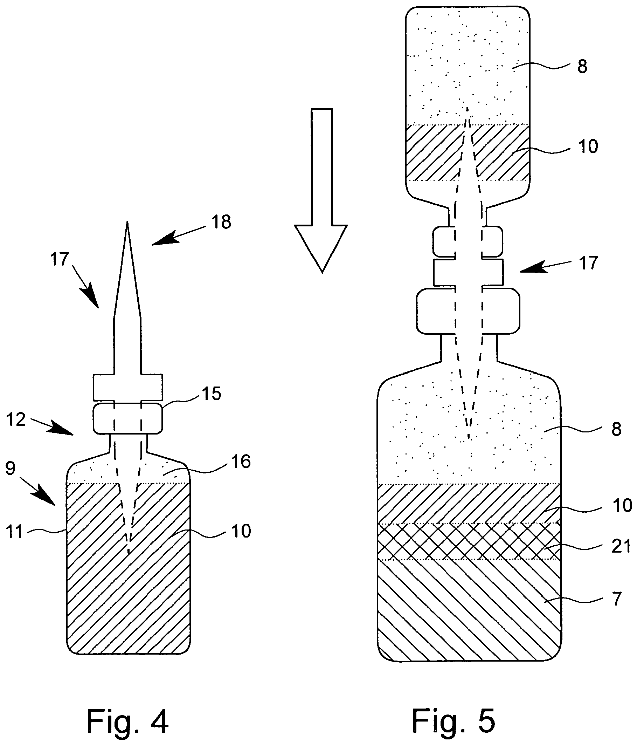

Vorzugsweise entspricht oder überschreitet das Volumen des Gases 8 das Volumen des zweiten Edukts 10. Insbesondere überschreitet das Volumen des Gases 8 in dem ersten Behältnis 1 das Volumen des zweiten Edukts 10 um mehr als 2 %, vorzugsweise mehr als 5 %, insbesondere mehr als 8 % und/oder weniger als 80 %, vorzugsweise weniger als 50 %, insbesondere weniger als 40 % oder 30 %. Hierdurch kann ein minimaler Platzbedarf des ersten Behältnisses 1 bei gleichzeitig homogener Herstellung des Impfstoffes 21 (vergleiche Figur 5) in dem ersten Behältnis 1 erreicht werden. Insbesondere verbleibt ein ausreichendes Volumen an Gas 8 in dem ersten Behältnis 1, wodurch ein homogenes Vermischen mittels Bewegung des ersten Behältnisses 1 erreicht werden kann.

Im Darstellungsbeispiel gemäß Figur 2 ist das zweite Behältnis 9 zumindest im Wesentlichen mit dem zweiten Edukt 10 angefüllt. Alternativ oder zusätzlich ist es jedoch auch möglich, dass das zweite Behältnis 9 vollkommen mit dem zweiten Edukt 10 aufgefüllt oder lediglich teilweise mit dem zweiten Edukt 10 gefüllt ist. Wenn lediglich eine teilweise Füllung des zweiten Behältnisses 9 mit dem zweiten Edukt 10 vorgesehen ist, kann ein Gas 16, insbesondere ein Schutzgas, vorgesehen sein, das vorzugsweise gemeinsam mit dem zweiten Edukt 10 das von der Wandung 11, insbesondere mit der Verschlusseinrichtung 14, umschlossene Volumen des zweiten Behältnisses 9 ausfüllt.

Es ist besonders bevorzugt, dass das Gesamtvolumen des ersten Behältnisses 1 das Volumen des ersten Edukts 7 mindestens um das Volumen des zweiten Edukts 10 übersteigt. Das Volumen des ersten Behältnisses 1 überschreitet also vorzugsweise die Summe der Volumina des ersten Edukts 7 und des zweiten Edukts 10.

Figur 3 zeigt eine Adaptereinrichtung 17 zur Herstellung einer fluidischen Verbindung zwischen dem ersten Behältnis 1 und dem zweiten Behältnis 9. Die Adaptereinrichtung 17 kann mindestens ein, bevorzugt zwei, aber auch mehr als zwei Adapterelemente 18 aufweisenden, insbesondere Nadeln, Hohlnadeln, Dorne, Keile oder dergleichen.

Die Adaptereinrichtung 17 weist vorzugsweise einen Fluidkanal 19 auf, insbesondere zur fluidischen Verbindung der Innenräume der Behältnisse 1 und 9. Die Adaptereinrichtung 17 ist im Darstellungsbeispiel als Doppeldorn bzw. Hohlnadel ausgebildet.

Die Adaptereinrichtung 17 kann derart ausgebildet sein, dass während des Transportes des zweiten Edukts 10 in das erste Behältnis 1 das Gas 8 bzw. Schutzgas aus dem ersten Behältnis 1 in das zweite Behältnis 9 gelangt, insbesondere transportiert wird. Hierzu kann die Adaptereinrichtung 17 eine Be- und/oder Entlüftungseinrichtung 20 aufweisen. Die Be- und/oder Entlüftungseinrichtung 20 ist vorzugsweise als Kanal ausgebildet, insbesondere zumindest im Wesentlichen parallel zu dem Fluidkanal 19. Es sind aber auch andere Lösungen möglich.

Bevorzugt weist die Adaptereinrichtung 17 einen Kanal als Be- und/oder Entlüftungseinrichtung 20 auf, dessen Öffnungen so angeordnet sind, dass bei ausflie-ßen des zweiten Edukts 10 aus dem zweiten Behältnis 9 unter Einfluss der Schwerkraft ein Rückstrom für das Gas 8 aus dem ersten Behältnis 1 in das zweite Behältnis 9 ermöglicht wird. Insbesondere kann die dem ersten Behältnis 1 zugewandte Öffnung der Be- und/oder Entlüftungseinrichtung 20 gegenüber der dem ersten Behältnis 1 zugewandten Öffnung des Fluidkanals 19 in Richtung der Längserstreckung der Adaptereinrichtung 17 zurückspringen bzw. auf der dem offenen Ende der Adaptereinrichtung 17 abgewandten Seite der Öffnung des Fluidkanals 19 angeordnet sein. Die dem zweiten Behältnis 9 zugewandte Öffnung der Be- und/oder Entlüftungseinrichtung 20 ist hingegen vorzugsweise näher am offenen Ende der Adaptereinrichtung 17 in Bezug auf die dem zweiten Behältnis 9 zugewandte Öffnung des Fluidkanals 19 angeordnet.

Die Adaptereinrichtung 17 im Darstellungsbeispiel gemäß Figur 3 ist einstückig ausgebildet. Es ist jedoch alternativ oder zusätzlich auch möglich, dass zwischen den Adapterelementen 18 ein Schlauch, ein Rohr, ein sonstiger flexibler oder steifer Übergang oder dergleichen angeordnet ist. Hierbei ist bevorzugt, dass der Fluidkanal 19 und/oder die Be- und/oder Entlüftungseinrichtung 20 ununterbrochen die Adapterelemente 18 verbindet. Insbesondere es möglich, dass zwischen den Adapterelementen 18 ein Schlauch, Rohr oder dergleichen vorgesehen, insbesondere angeformt ist. Dies ermöglicht eine flexible Handhabung und/oder Verlängerung der Adaptereinrichtung 17.

Die Adaptereinrichtung 17, insbesondere eines oder mehrere der Adapterelemente 18 kann bzw. können zu dem ersten Behältnis 1 und/oder zu dem zweiten Behältnis 9 spezifisch ausgebildet sein. Dies kann insbesondere dadurch realisiert werden, dass mindestens eines der Adapterelemente 18 mechanisch spezifisch zu einem der Behältnisse 1, 9, insbesondere zu einem dem jeweiligen Adapterelement 18 zugeordneten der Behältnisse 1, 9, ausgebildet ist, beispielsweise durch ein spezifisches Gewinde 3, 12, einen spezifischen Vorsprung, Hinterschnitt, eine spezifische Nase oder eine sonstige Strukturen. Hierdurch kann verhindert werden, dass andere Stoffe als das erste Edukt 7 und/oder das zweite Edukt 10 in das erste Behältnis 1 und/oder das zweite Behältnis 9 eingebracht werden. Ferner ist bevorzugt, das der Öffnungsbereich zumindest eines oder beider Behältnisse 1, 9 spezifisch für die Adaptereinrichtung 17, insbesondere für ein Adapterelement 18, ausgebildet ist.

Ferner ist bevorzugt, dass eine Verbindung lediglich zwischen den Behältnissen 1, 9, bevorzugt unter Ausschluss der umgebenden Atmosphäre hergestellt wird. Im Sinne der vorliegenden Erfindung gilt die Atmosphäre vorzugsweise bereits dann als ausgeschlossen, auch wenn ein geringes Restvolumen atmosphärischen Ursprungs in der Adaptereinrichtung 17 verbleibt, bespielsweise weniger als 10 ml oder 5 ml.

Das erste Edukt 7 und/oder das zweite Edukt 10 weisen vorzugsweise ein Vakzin auf. Ferner ist bevorzugt, dass die Edukte 7, 10 unterschiedlich sind, insbesondere unterschiedliche Vakzine aufweisen.

Wie in Figur 4 und 5 ersichtlich, kann mit der Adaptereinrichtung 17 eine Fluidverbindung zwischen dem ersten Behältnis 1 und dem zweiten Behältnis 9 hergestellt werden, wobei das zweite Edukt 10 bevorzugt unter Mitwirkung der Schwerkraft und/oder durch Ausübung von Druck auf das zweite Edukt 10 über die Wandung 11 des zweiten Behältnisses 9 in das erste Behältnis 1 transportiert werden kann, um dort mit dem ersten Edukt 7 den Impfstoff 21 zu bilden. Der Impfstoff 21 ist bevorzugt zur Prävention der Erkrankung von Schweinen durch Infektionen mit Mycoplasma hyopneumoniae und/oder Porcinem Circovirus, insbesondere Typ 2, vorzugsweise zur Prävention der Erkrankungen von Schweinen durch Infektionen mit Mycoplasma hyopneumoniae und Porcinem Circovirus Typ 2, ausgebildet, stellt also im Darstellungsbeispiel einen Kombiimpfstoff dar.

Das erste Behältnis 1, das zweite Behältnis 9 und vorzugsweise, die Adaptereinrichtung 17 bilden bevorzugt ein Kit bzw. Verbund, haben im Sinne der vorliegenden Erfindung also vorzugsweise mindestens eine verbindende Gemeinsamkeit. Diese Gemeinsamkeit kann darin bestehen, dass das erste Behältnis 1, das zweite Behältnis 9 und/oder die Adaptereinrichtung 17 gemeinsam verpackt sind. Alternativ oder zusätzlich ist es möglich, dass das erste Behältnis 1, das zweite Behältnis 9 und/oder die Adaptereinrichtung 17 eine gemeinsame Handlungsanweisung, einen gemeinsamen Beipackzettel, eine gemeinsame Herstellungsempfehlung oder dergleichen aufweisen. Auch ist es möglich, dass Beschriftungen, Etiketten Markierungen, Symbole oder sonstige Bezeichnungen der Behältnisse 1, 9 aufeinander verweisen oder dergleichen.

Das Kit kann optional auch weitere Komponenten aufweisen. Beispiele hierfür sind ein oder mehrere Injektionseinrichtungen 22, Adapter zum Anschluss des ersten Behältnisses 1 an eine Injektionseinrichtung 22, eine sonstige Einrichtung oder ein weiteres Behältnis.

Das Kit ist bevorzugt zur Herstellung eines Impfstoffes 21 zur Vorbeugung von Erkrankungen verursacht durch Infektionen mit Porcinem Circovirus Typ 2 und/oder Mycoplasma hyopneumoniae, vorugsweise zur Vorbeugung von Erkrankungen verursacht durch Infektionen mit Porcinem Circovirus Typ 2 und/oder Mycoplasma hyopneumoniae beim Schwein vorgesehen. Abweichende Anwendungen sind jedoch nicht ausgeschlossen. Insbesondere kann alternativ vorgesehen sein, andere als die genannten Vakzine oder sonstige Stoffe oder Stoffgemische als Edukte 7, 10 zu verwenden und/oder ähnliche Vorteile bezüglich der einfachen Handhabung und der verminderten Wahrscheinlichkeit eines Eintrags von Fremdstoffen, Krankheitserregern oder dergleichen zu erreichen. Insbesondere kann es sich bei den alternativen Edukten um ein oder mehrere Antigene der nachfolgenden Krankheitserreger handeln: Actinobacillus pleuropneumonia (A1); Adenovirus (A2); Alphavirus wie zum Beispiel Eastern Equine Encephalomyelitis Virus (A3); Bordetella bronchiseptica (A4); Brachyspira spp. (A5), vorzugsweise B. hyodyentheriae (A6); B. piosicoli (A7), Brucella suis, vorzugsweise der Biovaren 1, 2, und 3 (A8); Classical Swine Fever Virus (A9); Clostridium spp. (A10), vorzugsweise Cl. difficile (A11), Cl. perfringens der Typen A, B, und C (A12), Cl. novyi (A13), Cl. septicum (A14), Cl. tetani (A15); Coronavirus (A16), vorzugsweise Porcine Respiratory Corona Virus (A17); Eperythrozoonosis suis (A18); Erysipelothrix rhusiopathiae (A19) Escherichia coli (A20); Haemophilus parasuis, vorzugsweise der Subtypen 1, 7 and 14 (A21) Hemagglutinating Encephalomyelitis Virus (A22); Japanese Encephalitis Virus (A23); Lawsonia intracellularis (A24) Leptospira spp. (A25), vorzugsweise Leptospira australis (A26), Leptospira canicola (A27), Leptospira grippotyphosa (A28), Leptospira icterohaemorrhagicae (A29), Leptospira interrogans (A30), Leptospira pomona (A31), Leptospira hardjo (A32) und Leptospira tarassovi (A33); Mycobacterium spp. (A34) vorzugsweise M. avium (A35) und M. intracellulare (A36); Pasteurella multocida (A37); Porcine Cytomegalovirus (A38); Porcine Parvovirus (A39); Porcine Reproductive and Respiratory Syndrome (PRRS) Virus (A40); Pseudorabies Virus (A41); Rotavirus (A42); Salmonella spp. (A43), vorzugsweise S. thyphimurium (A44) und S. choleraesuis (A45); Staphylococcus spp. (A46) vorzugsweise Staph. hicus (A47); Streptococcus spp. (A48), vorzugsweise Strep. suis (A49); Swine Herpes Virus (A50); Swine Influenza Virus (A51); Swine Pox Virus (A52); Vesicular Stomatitis Virus (A53); Virus vom Vesicularem Exanthema vom Schwein (A54); und Mycoplasma hyosynoviae (A55). Vorzugsweise handelt es sich bei den Edukten 7 und 10 um unterschiedliche Antigene der hier aufgeführten Krankheitserreger, so dass der Impfstoff 21 nach Vermischung der verschiedenen Edukte zur Vorbeugung von Erkrankungen verursacht durch mindestens zwei der hier aufgeführten Krankheitserreger verwendet werden kann.

Zur Herstellung des Impfstoffes 21 kann, insbesondere wie in Figur 4 dargestellt, die Adaptereinrichtung 17 mit einem Adapterelement 18 durch die Verschlusseinrichtung 14 des zweiten Behältnisses 9 in dessen Innenraum eingeschoben werden. Die Verschlusseinrichtung 14 wird also vorzugsweise durch die Adaptereinrichtung 17, insbesondere ein Adapterelement 18 dieser, durchdrungen.

Weiter kann insbesondere, wie in Figur 5 dargestellt, der Verbund aus zweitem Behältnis 9 und Adaptereinrichtung 17, insbesondere mit einem weiteren Adapterelement 18 der Adaptereinrichtung 17, durch die Verschlusseinrichtung 5 des ersten Behältnisses 1 hindurch in dessen Innenraum eingeschoben werden. Hierbei ist bevorzugt, dass die Adaptereinrichtung 17 bzw. das Adapterelement 18 die Verschlusseinrichtung 5 des ersten Behältnisses 1 durchdringt. Hierdurch kann erreicht werden, dass mittels der Adaptereinrichtung 17 eine Fluidverbindung 20 zwischen den Innenräumen des ersten Behältnisses 1 und des zweiten Behältnisses 9 hergestellt wird.

In den Figuren 4 und 5 sind aus Gründen der Übersichtlichkeit Details der Öffnungsbereiche 3, 12 der Behältnisse 1, 9 sowie der Adaptereinrichtung 17 nicht dargestellt. Hierzu wird auf die Figuren 1 bis 3 verwiesen.

Wie in Figur 5 dargestellt, kann das zweite Edukt 10 durch die Adaptereinrichtung 17 in das erste Behältnis 1 transferiert werden, vorzugsweise unter Verwendung der Schwerkraft und/oder durch Kompression des bevorzugt flexibel ausgebildeten zweiten Behältnisses 9. Alternative und/oder zusätzliche Methoden bzw. Verfahrensweisen zum Transport des zweiten Edukts 10 in das erste Behältnis 1 sind ebenfalls möglich.

In einem weiteren, nicht dargestellten Beispiel wird die Adaptereinrichtung 17 zuerst dazu verwendet, die Verschlusseinrichtung 5 des ersten Behältnisses 1 zu durchdringen. In einem zweiten Schritt wird dann das zweite Behältnis 9 auf die bereits in das erste Behältnis 1 eingesetzte Adaptereinrichtung 17 aufgesteckt oder auf sonstige Weise derartig mit der Adaptereinrichtung 17 in Verbindung gebracht, dass eine fluidische Verbindung 20 zwischen dem ersten Behältnis 1 und dem zweiten Behältnis 9 hergestellt wird. In jedem Falle ist bevorzugt, nach Einbringen der Adaptereinrichtung 17 in ein erstes der Behältnisse 1, 9, das noch nicht mit der Adaptereinrichtung 17 verbundene der Behältnisse 1, 9 auf den aufrecht stehenden Verbund aus Adaptereinrichtung 17 und dem anderen der Behältnisse 1, 9 aufgesteckt wird. Hierdurch kann ein Ausfließen eines der Edukte 7, 10 verhindert werden.

Ein Aspekt der vorliegenden Erfindung betrifft das erstmalige und/oder einmalige Durchdringen mindestens einer der Verschlusseinrichtungen 5, 14 mittels der Adaptereinrichtung 17. Besonders bevorzugt weisen beide Behältnisse 1, 9 Verschlusseinrichtungen 5, 14 auf, die jeweils erstmalig und/oder einmalig durchdrungen, durchstochen bzw. perforiert werden. Dies wird im Darstellungsbeispiel, dadurch ermöglicht, dass das erste Behältnis 1 lediglich teilweise mit dem ersten Edukt 7 gefüllt ist. In vorteilhafter Weise ist es hierdurch möglich, den Impfstoff 21, insbesondere Kombiimpfstoff, herzustellen, ohne dass mit mehreren Adaptereinrichtungen 17 bzw. mit mehr als zwei Behältnissen 1, 9 gearbeitet werden muss. Hieraus resultiert eine einfache, sichere und eindeutige Handhabung, bei der zur Herstellung des Impfstoffes 21 nur eine Adaptereinrichtung 17 verwendet werden muss, wodurch Verwechslungen mit anderen Edukten sowie dem Eintrag von Verunreinigungen oder Krankheitserregern wirksam vorgebeugt wird.

Diese positiven Eigenschaften können dadurch verstärkt werden, dass das erste Behältnis 1, gegebenenfalls mit der Adaptereinrichtung 17, dazu ausgebildet ist, unmittelbar in der Injektionseinrichtung 22, insbesondere in einer Injektionspistole, in einem Druckinjektor und/oder in einer Selbstfüllerspritze (vgl. Figur 7) oder hiermit eingesetzt zu werden. Das erste Behältnis 1 kann also in oder mit einer vorzugsweise mehrfach verwendbaren Injektionseinrichtung 22 eingesetzt werden. Hierdurch kann erreicht werden, dass jede Verschlusseinrichtung 5, 14 lediglich erstmalig und einmalig durchstoßen bzw. durchbrochen werden muss. Dies ermöglicht ein Höchstmaß an Anwendungssicherheit, einfacher Handhabung sowie Hygiene.

Es kann vorgesehen sein, dass das erste Behältnis 1 zum Einsatz in oder mit einer Injektionseinrichtung 22 in einem Hals- bzw. Mündungsbereich, insbesondere im Öffnungsbereich 3, zur Verbindung mit einer insbesondere mehrfach verwendbaren Injektionseinrichtung 22 ausgebildet ist. Die Injektionseinrichtung 22 kann eine Aufnahme 23 für das erste Behältnis 1 aufweisen. Hierbei kann vorgesehen sein, dass die Längserstreckung und/oder der Durchmesser des ersten Behältnisses 1 für den Einsatz in einer, insbesondere mehrfach verwendbaren, Injektionseinrichtung 22 ausgebildet ist. Beispielsweise kann das erste Behältnis 1 einen Durchmesser größer als 1 cm, vorzugsweise größer als 2 cm, insbesondere größer als 3 cm und/oder geringer als 10 cm, vorzugsweise geringer als 8 cm, insbesondere geringer als 6 cm aufweisen. Alternativ oder zusätzlich ist es möglich, dass das erste Behältnis 1 eine Längserstreckung aufweist, die größer als 3 cm, vorzugsweise größer als 5 cm, insbesondere größer als 6 cm und/oder geringer als 30 cm, vorzugsweise geringer als 25 cm, insbesondere geringer als 20 cm ist.

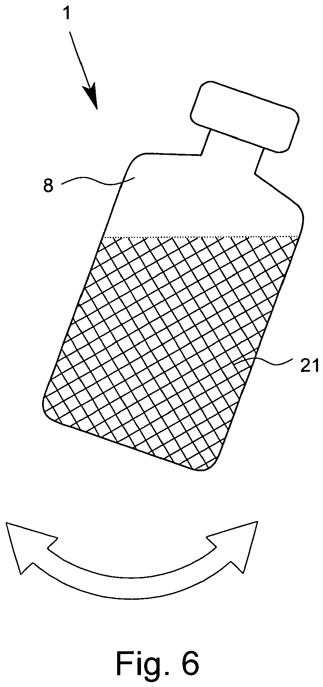

Sobald das zweite Edukt 10 durch die Adaptereinrichtung 17 in das erste Behältnis 1 gelangt ist, kann Impfstoff 21 gebildet werden. Hierbei ist es möglich, dass der Impfstoff 21 durch Mischung der Edukte 7, 10, durch Lösung der Edukte 7, 10 ineinander und/oder durch Reaktion der Edukte 7, 10 oder Teile hiervon mit einander erzeugt wird. Es ist bevorzugt, dass, wie in Figur 6 mit dem Pfeil angedeutet, das erste Behältnis 1 mit den Edukten 7, 10 in Bewegung versetzt wird, insbesondere geschüttelt, geschwenkt, in Rotation versetzt wird oder dergleichen. Hierdurch kann eine homogene und/oder beschleunigte Durchmischung, Reaktion und/oder Lösung erreicht oder begünstigt werden.

Im Darstellungsbeispiel weist das erste Edukt 7 und/oder das zweite Edukt 10 Vakzin vorzugsweise in Mengen größer als 30 Gewichtsprozent, vorzugsweise größer als 40 Gewichtsprozent, insbesondere größer als 50 Gewichtsprozent; und/oder geringer als 90 Gewichtsprozent, vorzugsweise geringer als 80 Gewichtsprozent, insbesondere geringer als 70 Gewichtsprozent auf. Ferner kann das erste Edukt 7, das zweite Edukt 10, und/oder der hierdurch gebildete Impfstoff 21 eine Suspension sein, vorzugsweise mit Vakzinen und/oder unlöslichen Proteinen, vorzugsweise in Mengen größer als 30 Gewichtsprozent, vorzugsweise größer als 40 Gewichtsprozent, insbesondere größer als 50 Gewichtsprozent; und/oder geringer als 90 Gewichtsprozent, vorzugsweise geringer als 80 Gewichtsprozent, insbesondere geringer als 70 Gewichtsprozent. Hierdurch wird eine Wirksamkeit bei geringen Impfstoffmengen und gleichzeitig ausreichend geringer Viskosität erreicht, was die Applizierbarkeit begünstigt.

Das zweite Edukt 10 weist vorzugsweise lediglich die andere der Komponenten Mycoplasma Vakzin bzw. Mycoplasma Antigen und Circovirus Vakzin bzw. Circovirus Antigen auf. Es ist also bevorzugt, dass, wenn das erste Edukt 7 Mycoplasma Vakzin bzw. ein oder mehrere Mycoplasma Antigene aufweist, das erste Edukt 7 kein Circovirus Vakzin bzw. kein Circovirus Antigen aufweist, dafür jedoch das zweite Edukt 10 Circovirus Vakzin bzw. ein oder mehrere Circovirus Antigene aufweist, nicht hingegen Mycoplasma Vakzin bzw. Mycoplasma Antigen. Es ist jedoch für keines der Edukte 7, 10 ausgeschlossen, dass das jeweilige Edukt nicht noch zusätzliche Stoffe aufweist. Hierzu können Wasser, Hilfsstoffe, Adjuvantien, Konservierungsmittel oder dergleichen zählen.

In einem Beispiel beträgt das Volumenverhältnis des ersten Edukts 7 zu dem zweiten Edukt 10 3:1 bis 1:3, vorzugsweise 2:1 bis 1:2, insbesondere etwa 1:1. Das erste Edukt 7 und/oder das zweite Edukt 10 und/oder der Impfstoff 21 kann eine Viskosität aufweisen, die weniger als 10.000 mPa·s, vorzugsweise weniger als 1000 mPa·s, insbesondere weniger als 500 mPa·s; und/oder mehr als 5 mPa·s, vorzugsweise mehr als 10 mPa·s, insbesondere mehr als 20 mPa·s beträgt, und zwar gemessen mit einem Brookfield Viskosimeter nach EN ISO 2555 bei 5 °C. Vorzugsweise sind das erste Edukt 7 und/oder das zweite Edukt 10 bei 2 °C bis 8 °C flüssig und/oder weisen einen Schmelzpunkt auf, der geringer als 1 °C, vorzugsweise geringer als 0 °C, insbesondere geringer als -0,2 °C und/oder höher als -1,5 °C, vorzugsweise höher als -1 °C, insbesondere höher als -0,8 °C ist.

Gemäß einem weiteren Aspekt der vorliegenden Erfindung ist bevorzugt, dass der Impfstoff 21 eine charakteristische und von den beiden Edukten 7, 10 verschiedene Farbe aufweist. Die spezifische Farbe des Impfstoffs kann beispielsweise aus der Mischung der verschieden gefärbten Edukte 7 und 10 entstehen. Beispielswese führt die Mischung eines gelb gefärbten Edukts 7 mit einem rot gefärbten Edukt 10 zu einer orangen Färbung des Impfstoffs 21. Es ist möglich, dass Farbstoffkomponenten in den Edukten 7, 10 bei Herstellung des Impfstoffs 21 die charakteristische Farbe durch Reaktion oder sonstige Wechselwirkungen erzeugen. Alternativ oder zusätzlich kann vorgesehen sein, dass der Impfstoff 21 im Gegensatz zu mindestens einem der Edukte 7, 10 eine oder keine Farbe aufweist. Hierdurch kann anhand der charakteristischen Farbe sichergestellt werden, dass der Impfstoff 21 erfolgreich hergestellt worden ist. Entsprechendes ist auch möglich, wenn zumindest eines der Edukte eine Farbe aufweist, der Impfstoff 21 nach seiner Herstellung jedoch nicht mehr.

Wie bereits oben ausgeführt, kann das Mycoplasma Vakzin attenuierte und/oder inaktivierte Bakterien, Fragmente von Bakterien oder rekombinant herstellte Teile von Mycoplasma hyopneumoniae, zumindest aber ein oder mehrere Mycoplasma hyopneumoniae Antigene aufweisen. Vorzugsweise, stammt das Mycoplasma hyopneumoniae Antigen von dem Mycoplasma hyopneumoniae J-Stamm ab bzw. handelt es sich bei dem inaktivierten Mycoplasma hyopneumoniae Bakterien um solche des J-Stamms. Weiterhin kann es sich bei dem Mycoplasma Vakzin um einen der nachfolgenden Vakzine handeln bzw. kann es sich bei dem Mycoplasma hyopneumoniae Antigen um das oder die Antigene handeln, welche in einem der nachfolgenden Vakzinen enthalten sind: Ingelvac®MycoFlex (Boehringer Ingelheim Vetmedica Inc, St Joseph, MO, USA), Porcilis M. hyo, Myco Silencer® BPM, Myco Silencer® BPME, Myco Silencer® ME, Myco Silencer® M, Myco Silencer® Once, Myco Silencer® MEH (alle von Intervet Inc., Millsboro, USA) Stellamune Mycoplasma (Pfizer Inc., New York, NY, USA), Suvaxyn Mycoplasma, Suvaxyn M. hyo, Suvaxyn MH-One (alle ehemals Fort Dodge Animal Health, Overland Park, KS, USA (jetzt Pfizer Animal Health).

Das Circovirus Vakzin kann attenuiertes und/oder inaktiviertes porcines Circovirus, vorzugsweise Typ 2, insbesondere das ORF2 Protein von Typ 2 aufweisen. Besonders bevorzugt ist die Verwendung von recombinant exprimierten ORF2 Protein des Prorcinen Circovirus Typ 2, vorzugsweise exprimiert in und/oder gewonnen aus in vitro Zellkultur. Beispiele von ORF2 Proteinen des Procinen Circovirus Typ 2 sind unter anderem in der internationalen Patentanmeldung WO2006-072065 beschrieben, Diese haben sich als besonders vorteilhaft für eine effektive Impfung herausgestellt. Weiterhin kann es sich bei dem Circovirus Vakzin um einen der nachfolgenden Impfstoffe handeln, bzw. kann es sich bei dem Circovirus Antigen um das oder die Antigene handeln, welche in einem der nachfolgenden Vakzinen enthalten sind: Ingelvac®CircoFLEX, (Boehringer Ingelheim Vetmedica Inc, St Joseph, MO, USA), CircoVac® (Merial SAS, Lyon, France), CircoVent (Intervet Inc., Millsboro, DE, USA), or Suvaxyn PCV-2 One Dose® (Fort Dodge Animal Health, Kansas City, KA, USA).

Das Circovirus Vakzin, sofern es das ORF2 Protein enthält, beinhaltet vorzugsweise zwischen 2 µg und 150 µg, vorzugsweise zwischen 2 µg und 60 µg, weiterhin bevorzugt zwischen 2 µg und 50 µg, weiterhin bevorzugt zwischen 2µg und 40 µg, weiterhin bevorzugt zwischen 2 µg und 30 µg, weiterhin bevorzugt zwischen 2µg und 25 µg, weiterin bevorzugt zwischen 2µg und 20 µg, weiterhin bevorzugt zwischen 4 µg und 20 µg, weiterhin bevorzugt zwischen 4µg und 16 µg ORF2 Protein pro zu verabreichenden Dosis auf. Das Circovirus Vakzin ist vorzugsweise so hergestellt bzw. hergerichtet, dass 1 ml des Vakzins einer Dosis von 1 entspricht.

Das Mycoplasma Vakzin, sofern es inaktivierte Mycoplasma Bakterien, vorzugsweise inaktivierte Mycoplasma hyopneumoniae Bakterien enthält, beinhaltet vorzugsweise zwischen 10³ und 10⁹ colony forming units (CFU), vorzugsweise zwischen 10⁴ und 10⁸ (CFU), weiterhin bevorzugt zwischen 10⁵ und 10⁶ (CFU) pro zu verabreichenden Dosis, wobei die entsprechende CFU vor Inaktivierung der Bakterien eingestellt wird. Das Mycoplasma Vakzin ist vorzugsweise so hergestellt bzw. hergerichtet, dass 1 ml des Vakzins einer Dosis von 1 entspricht.

Das Mycoplasma Vakzin kann vorzugsweise attenuierte und/oder inaktivierte Bakterien oder Fragmente von Bakterien des Stamms Mycoplasma hyopneumoniae bzw. korrespondierende Antigene aufweisen, vorzugsweise in Mengen größer als 30 Gewichtsprozent, vorzugsweise größer als 40 Gewichtsprozent, insbesondere größer als 50 Gewichtsprozent; und/oder geringer als 90 Gewichtsprozent, vorzugsweise geringer als 80 Gewichtsprozent, insbesondere geringer als 70 Gewichtsprozent.

Das Circovirus Vakzin kann vorzugsweise attenuiertes und/oder inaktiviertes Porcines Circovirus, vorzugsweise Typ 2 , insbesondere Porcines Circovirustyp 2 ORF2 Protein bzw. korrespondierende Antigene aufweisen, vorzugsweise in Mengen größer als 30 Gewichtsprozent, vorzugsweise größer als 40 Gewichtsprozent, insbesondere größer als 50 Gewichtsprozent; und/oder geringer als 90 Gewichtsprozent, vorzugsweise geringer als 80 Gewichtsprozent, insbesondere geringer als 70 Gewichtsprozent.

Ferner kann mindestens eines der Edukte 7, 10 und/oder der Impfstoff 21 ein, vorzugsweise polymeres, Adjuvans, insbesondere Carbomer, aufweisen. Vorzugsweise enthält eines der beiden Edukte oder weiterhin bevorzugt beide Edukte eine Adjuvanzmenge von 500 µg bis 5 mg, vorzugsweise von 750 µg bis 2,5 mg, weiterhin bevorzugt von ungefähr 1 mg pro zu verabreichenden Dosis auf Die Edukte sind vorzugsweise so hergestellt bzw. hergerichtet, dass 1 ml des jeweiligen Edukts einer Dosis von 1 entspricht. Insbesondere kann also mindestens eines der Edukte 7, 10 ein oder mehrere Adjuvanzien einer Gesamtmenge von mehr als 500 µg/ml, vorzugsweise mehr als 750 µg/ml und/oder weniger als 5 mg/ml, vorzugsweise weniger als 2,5 mg/ml aufweisen.

Auch ist es möglich, dass mindestens eines der Edukte 7, 10 und/oder der Impfstoff 21 ein Adjuvans in Mengen größer als 0,1 Gewichtsprozent, vorzugsweise größer als 1 Gewichtsprozent, insbesondere größer als 2 Gewichtsprozent; und/oder weniger als 20 Gewichtsprozent, vorzugsweise weniger als 10 Gewichtsprozent, insbesondere weniger als 5 Gewichtsprozent aufweist. Auch ist es möglich, dass in dem Impfstoff 21 ein vorzugsweise polymeres Adjuvans, insbesondere Carbomer, gebildet wird.

Das Mycoplasma Vakzin und/oder das Circovirus Vakzin bzw. zumindest eines der Edukte 7, 10, und/oder der Impfstoff 21 kann Formaldehyd aufweisen, vorzugsweise mit einer Konzentration geringer als 2,5 mg/m³, vorzugsweise weniger als 1,5 mg/m³, insbesondere 0,74 mg/m³ oder weniger. Alternativ oder zusätzlich kann zumindest eines der Edukte 7, 10, vorzugsweise beide Edukte 7, 10, Wasser in einer Konzentration von mindestens 20 Gewichtsprozent, vorzugsweise mindestens 30 Gewichtsprozent, insbesondere mindestens 40 Gewichtsprozent; und/oder höchstens 80 Gewichtsprozent, vorzugsweise höchstens 70 Gewichtsprozent, insbesondere höchstens 60 Gewichtsprozent aufweisen.

Das erste Edukt 7, das zweite Edukt 10 und/oder der Impfstoff 21 kann einen oder mehrere Hilfsstoffe aufweisenden, insbesondere ein Konservierungsmittel, ein Antioxidanz und/oder einen Emulgator, und zwar vorzugsweise jeweils in einer Konzentration von mindestens 0,1 Gewichtsprozent, vorzugsweise mindestens 0,2 Gewichtsprozent, insbesondere mindestens 0,3 Gewichtsprozent; und/oder höchstens 10 Gewichtsprozent, vorzugsweise höchstens 5 Gewichtsprozent, insbesondere höchstens 3 Gewichtsprozent.

Die bevorzugte Anwendung der vorliegenden Erfindung bezieht sich auf die Herstellung des Impfstoffes 21 aus zwei Vakzinen, besonders bevorzugt auf die Herstellung eines Impfstoffes 21 zur Verwendung bei der insbesondere gleichzeitigen Immunisierung gegen die Erkrankung Porcine Circovirus Disease "PCVD" und/oder Enzootische Pneumonie "EP" bzw. gegen die Infektion mit Porcinem Circovirus und/oder mit Mycoplasma Bakterien, vorzugsweise zur Verwendung bei der Immunisierung gegen die Erkrankung Porcine Circovirus Disease "PCVD" und Enzootische Pneumonie "EP" bzw. zur gegen die Infektion mit Porcinem Circovirus, insbesonder Typ 2 und mit Mycoplasma Bakterien, insbesondere Mycoplasma hyopneumoniae. Ferner bezieht sich die vorliegende Erfindung primär auf Impfstoffe für die Veterinärmedizin, insbesondere zur Anwendung beim Schwein.

Die vorliegende Erfindung ist jedoch nicht beschränkt auf diese Kombination. Insbesondere ist es möglich, das vorschlagsgemäße Kit auch mit anderen Edukten, insbesondere Vakzinen oder Antigenen, wie beispielsweise mit Antigen der oben beschriebenen Krankheitserreger zu verwenden. Auch in diesem Fall kann es möglich sein, zumindest in Bezug auf die einfache und besonders hygienische Anwendbarkeit ähnliche Vorteile zu erzielen. Entsprechendes gilt auch für den Aspekt der vorliegenden Erfindung, dass der hergestellte Impfstoff 21 eine von den Edukten 7, 10 unterschiedliche Farbe aufweist, wodurch eine wirksame, einfache und effektive Kontrolle über die erfolgreiche Herstellung des Impfstoffs 21 ermöglicht wird. Die spezifische Farbe des Impfstoffs kann beispielsweise aus der Mischung der verschieden gefärbten Edukte 7 und 10 entstehen. Beispielsweise führt die Mischung eines gelb gefärbten Edukts 7 mit einem rot gefärbten Edukt 10 zu einer orangen Färbung des Impfstoffs 21. Auch die Verwendbarkeit des ersten Behältnisses 1 mit einer Injektionseinrichtung 22 kann auch in anderen Bereichen erfolgreich und vorteilhaft angewendet werden, wobei insbesondere ähnliche Vorteile in Bezug auf verbesserte Hygiene und/oder verminderten Materialeinsatz möglich sind. Besonders bevorzugt ist jedoch zumindest eine Kombination des Vorschlagsgemäßen Kits mit einer charakteristischen Farbe des hergestellten Impfstoffes 21, die von den Farben der Edukte 7, 10 abweicht. Dies führt zu einem ausgesprochen hohen Grad an Anwendungssicherheit. Die einzelnen Aspekte der vorliegenden Erfindung können jedoch auch in sonstiger, beliebiger Weise kombiniert werden.

### Bezugszeichen:

- 1: Erstes Behältnis
- 2: Wandung
- 3: Öffnungsbereich
- 4: Flansch
- 5: Verschlusseinrichtung
- 6: Klemmelement
- 7: Erstes Edukt
- 8: Gas
- 9: Zweites Behältnis
- 10: Zweites Edukt
- 11: Wandung
- 12: Öffnungsbereich
- 13: Flansch
- 14: Verschlusseinrichtung
- 15: Klemmelement
- 16: Gas
- 17: Adaptereinrichtung
- 18: Adapterelement
- 19: Fluidkanal
- 20: Be- und/oder Entlüftungseinrichtung
- 21: Impfstoff
- 22: Injektionseinrichtung
- 23: Aufnahme
- 24: Lebewesen

## Patentansprüche

1. Kit zur Herstellung eines Impfstoffes (21) zur Verwendung bei der Immunisierung gegen die Erkrankungen Porcine Circovirus Disease "PCVD" und Enzootische Pneumonie "EP", wobei das Kit ein erstes Edukt (7), ein vom ersten verschiedenes zweites Edukt (10), ein erstes, mit dem ersten Edukt (7) lediglich teilweise gefülltes Behältnis (1), ein zweites, das zweite Edukt (10) aufweisendes Behältnis (9) und eine Adaptereinrichtung (17) zur Herstellung einer Fluidverbindung zwischen dem ersten und zweiten Behältnis (1, 9) aufweist,
wobei die Behältnisse (1, 9)jeweils als Flasche ausgebildet sind,
wobei mindestens eines der Behältnisse (1, 9) werkseitig mit einer Verschlusseinrichtung (5) verschlossen ist und das zweite Behältnis (9) mittels der Adaptereinrichtung (17) unter erstmaligem und/oder lediglich einmaligem Durchbrechen der Verschlusseinrichtung (5) derart mit dem ersten Behältnis (1) verbindbar ist, dass das zweite Edukt (10) in das erste Behältnis (1) gelangt und dort mit dem ersten Edukt (7) den Impfstoff (21) bildet,
wobei beide Edukte (7, 10) bei 2 °C bis 8 °C flüssig sind und/oder einen Schmelzpunkt aufweisen, der geringer als 1 °C ist, wobei das erste Edukt (7) lediglich eine erste der Komponenten Mycoplasma Vakzin und Circovirus Vakzin aufweist, das zweite Edukt (10) lediglich die andere der Komponenten Mycoplasma Vakzin und Circovirus Vakzin aufweist, und, zur homogenen Herstellung des Impfstoffs (21) in dem ersten Behältnis (1), das Gesamtvolumen des ersten Behältnisses (1) das Volumen des ersten Edukts (7) mindestens um das Volumen des zweiten Edukts (10) übersteigt.

2. Kit nach Anspruch 1, **dadurch gekennzeichnet, dass** das erste Edukt (7), das zweite Edukt (10) und/oder der Impfstoff (21) eine Suspension ist, vorzugsweise mit Vakzinen und/oder unlöslichen Proteinen, vorzugsweise in Mengen größer als 30 Gew.-%, vorzugsweise größer als 40 Gew.-%, insbesondere größer als 50 Gew.-%; und/oder geringer als 90 Gew.-%, vorzugsweise geringer als 80 Gew.-%, insbesondere geringer als 70 Gew.-%.

3. Kit nach einem der voranstehenden Ansprüche, **dadurch gekennzeichnet, dass** das Volumenverhältnis des ersten Edukts (7) zum zweiten Edukt (10) 3:1 bis 1:3, vorzugsweise 2:1 bis 1:2, insbesondere etwa 1:1 beträgt.

4. Kit nach einem der voranstehenden Ansprüche, **dadurch gekennzeichnet, dass** das erste Edukt (7) und/oder das zweite Edukt (10) und/oder der Impfstoff (21) eine Viskosität aufweist, die geringer als 10000 mPa·s, vorzugsweise geringer als 1000 mPa·s, insbesondere geringer als 500 mPa·s; und/oder höher als 5 mPa·s, vorzugsweise höher als 10 mPa·s, insbesondere höher als 20 mPa·s ist, gemessen mit einem Brookfield-Viskosimeter nach EN ISO 2555 bei 5 °C.

5. Kit nach einem der voranstehenden Ansprüche, **dadurch gekennzeichnet, dass** der Impfstoff (21) eine charakteristische und von beiden Edukten (7, 10) verschiedene Farbe aufweist; und/oder dass der Impfstoff (21) im Gegensatz zu mindestens einem der Edukte (7, 10) eine oder keine Farbe aufweist.

6. Kit nach einem der voranstehenden Ansprüche, **dadurch gekennzeichnet, dass** mindestens eines der Edukte (7, 10) und/oder der Impfstoff (21) ein vorzugsweise polymeres Adjuvans, insbesondere Carbomer, aufweist, und/oder
dass in dem Impfstoff (21) ein vorzugsweise polymeres Adjuvans, insbesondere Carbomer, gebildet wird; und/oder
dass das Mycoplasma Vakzin und/oder das Circovirus Vakzin Formaldehyd mit einer Konzentration geringer als 2,5 mg/m³, vorzugsweise geringer als 1,5 mg/m³, insbesondere 0.74 mg/m³ oder weniger aufweist.

7. Kit nach einem der voranstehenden Ansprüche, **dadurch gekennzeichnet, dass** das erste und/oder zweite Edukt (7, 10) Wasser in einer Konzentration von mindestens 20 Gew.-%, vorzugsweise mindestens 30 Gew.-%, insbesondere mindestens 40 Gew.-%; und/oder höchstens 80 Gew.-%, vorzugsweise höchstens 70 Gew.-%, insbesondere höchstens 60 Gew.-% aufweist; und/oder
dass das erste Edukt (7) und/oder zweite Edukt (10) ein oder mehrere Hilfsstoffe, insbesondere Konservierungsmittel, Antioxidanzien und/oder Emulgator, aufweist.

8. Kit nach einem der voranstehenden Ansprüche, **dadurch gekennzeichnet, dass** das erste Behältnis (1) mit dem ersten Edukt (7) zu weniger als 70 %, vorzugsweise weniger als 50 % , insbesondere weniger als 45 % gefüllt ist; und/oder
dass das erste Behältnis (1) Gas (8), vorzugsweise Schutzgas, aufweist, vorzugsweise wobei das Volumen des Gases (8) mindestens dem Volumen des zweiten Edukts (10) in dem zweiten Behältnis (9) entspricht, insbesondere wobei das Volumen des Gases (8) in dem ersten Behältnis (1) das Volumen des zweiten Edukts (10) um mehr als 2 %, vorzugsweise mehr als 5 %, insbesondere mehr als 8 % und/oder weniger als 80 %, vorzugsweise weniger 50 %, insbesondere weniger als 40 % oder 30 % übersteigt.

9. Kit nach Anspruch 8, **dadurch gekennzeichnet, dass** die Adaptereinrichtung (17) derart ausgebildet ist, dass während des Transportes des zweiten Edukts (10) in das erste Behältnis (1) das Gas (8) bzw. Schutzgas aus dem ersten Behältnis (1) in das zweite Behältnis (9) gelangt, insbesondere transportiert wird; und/oder
dass das zweite Edukt (10) unter Einfluss der Schwerkraft in das erste Behältnis (1) gelangt.

10. Kit nach einem der voranstehenden Ansprüche, **dadurch gekennzeichnet, dass** die Adaptereinrichtung (17) zum fluidischen Verbinden der von den Behältnissen (1, 9) gebildeten Innenräume ausgebildet ist, wobei die Adaptereinrichtung (17) einen Fluidkanal (19) zum Herstellen der Fluidverbindung zwischen den von den Behältnissen (1, 9) gebildeten Innenräumen aufweist, vorzugsweise wobei die Adaptereinrichtung (17) einen Kanal zur Be- bzw. Entlüftung aufweist, der zusätzlich zu dem Fluidkanal (19) der Adaptereinrichtung (17) vorgesehen ist, insbesondere parallel hierzu.

11. Kit nach Anspruch 10, **dadurch gekennzeichnet, dass** eine Öffnung des Kanals zur Be- bzw. Entlüftung in Bezug auf die Auslassöffnung des Fluidkanals (19) auf der dem ersten Behältnis (1) zugewandten Seite in Richtung der Haupterstreckung der Adaptereinrichtung (17) zurückgesetzt ist, und/oder
dass eine dem zweiten Behältnis (9) zugewandte Öffnung des Kanals zur Be- bzw. Entlüftung auf der dem zweiten Behältnis (9) zugewandten, offenen Ende der Adaptereinrichtung (17) zugewandten Seite der dem zweiten Behältnis (9) zugewandten Auslassöffnung des Fluidkanals (19) angeordnet ist.

12. Kit nach einem der voranstehenden Ansprüche, **dadurch gekennzeichnet, dass** die Adaptereinrichtung (17) zwei einen Fluidkanal (19) und/oder eine Be- bzw. Entlüftungseinrichtung bildende, den Behältnissen (1, 9) zugeordnete Adapterelemente (18) aufweist, vorzugsweise ausgebildet als Transfernadel, Hohlnadel und/oder Dorn; und/oder
dass die Adaptereinrichtung (17) zu dem ersten Behältnis (1) und/oder zweiten Behältnis (9) spezifisch, vorzugsweise mechanisch spezifisch, ausgebildet ist, vorzugsweise wobei das Einbringen anderer Stoffe als das erste und/oder zweite Edukt (7, 10) in das erste und/oder zweite Behältnis (1, 9) verhindert wird; und/oder
dass die Adaptereinrichtung (17) dazu ausgebildet ist, eine Verbindung lediglich zwischen dem ersten und zweiten Behältnis (1, 9), insbesondere unter Ausschluss der umgebenden Atmosphäre, herzustellen

13. Kit nach einem der voranstehenden Ansprüche, **dadurch gekennzeichnet, dass** für die Darreichungsform das erste Behältnis (1), das zweite Behältnis (9) sowie die Adaptereinrichtung (17) einen geschlossenen Verbund bilden, vorzugsweise mit einer gemeinsamen Verpackung.

14. Verwendung eines Kits gemäß einem der voranstehenden Ansprüche, zur Herstellung und/oder Bereitstellung eines Impfstoffs (21) zur gleichzeitigen Immunisierung gegen die Erkrankungen Porcine Circovirus Disease "PCVD" und Enzootische Pneumonie "EP".

15. Verfahren zur Bereitstellung eines Impfstoffes (21) aus einem ersten Edukt (7) und mit einem zweiten, vom ersten verschiedenen Edukt (10), zur gleichzeitigen Immunisierung gegen die Erkrankungen Porcine Circovirus Disease "PCVD" und Enzootische Pneumonie "EP" mit einem Kit nach einem der Ansprüche 1 bis 13, wobei zwischen dem mit dem ersten Edukt (7) lediglich teilweise gefüllten Behältnis (1) und einem zweiten Behältnis (9) durch die Adaptereinrichtung (17) eine Fluidverbindung derart hergestellt wird, dass mittels der Adaptereinrichtung (17) unter erstmaligem und/oder lediglich einmaligem Durchbrechen mindestens einer werkseitig vorgesehenen Verschlusseinrichtung (14) mindestens eines der Behältnisse (1, 9) eine Fluidverbindung zwischen dem ersten und zweiten Behältnis (1, 9) hergestellt wird, wobei das zweite Edukt (10) durch die Adaptereinrichtung (17) und die Verschlusseinrichtung (5) hindurch in das erste Behältnis (1) befördert wird, und wobei in dem ersten Behältnis (1) aus dem ersten und zweiten Edukt (7, 10) der Impfstoff (21) gebildet wird.

## Claims

1. Kit for preparing a vaccinating agent (21) for use in immunization against the diseases Porcine Circovirus Disease "PCVD" and Enzootic Pneumonia "EP", said kit comprising a first starting material (7), a second starting material (10) different from the first, a first container (1) only partly filled with the first starting material (7), a second container (9) comprising the second starting material (10) and an adapter device (17) for providing a fluid connection between the first and second container (1, 9),
wherein the containers (1, 9) are each designed as a bottle,
wherein at least one of the containers (1, 9) is closed at the factory with a closure device (5) and the second container (9) is connectable to the first container (1) by means of the adapter device (17) by breaking through the closure device (5) for the first and/or only time, such that the second starting material (10) enters the first container (1) and there forms the vaccinating agent (21) with the first starting material (7),
wherein both starting materials (7, 10) are liquid at 2 °C to 8 °C and/or have a melting point of less than 1 °C, wherein the first starting material (7) comprises only a first of the components Mycoplasma vaccine and Circovirus vaccine, the second starting material (10) comprises only the other of the components Mycoplasma vaccine and Circovirus vaccine, and, for homogeneous production of the vaccinating agent (21) in the first container (1), the total volume of the first container (1) exceeds the volume of the first starting material (7) at least by the volume of the second starting material (10).

2. Kit according to claim 1, **characterized in that** the first starting material (7), the second starting material (10) and/or the vaccinating agent (21) is a suspension, preferably with vaccines and/or insoluble proteins, preferably in amounts of more than 30 % by weight, preferably more than 40 % by weight, in particular more than 50 % by weight; and/or less than 90 % by weight, preferably less than 80 % by weight, in particular less than 70 % by weight.

3. Kit according to one of the preceding claims, **characterized in that** the ratio by volume of the first starting material (7) to the second starting material (10) is 3:1 to 1:3, preferably 2:1 to 1:2, in particular about 1:1.

4. Kit according to one of the preceding claims, **characterized in that** the first starting material (7) and/or the second starting material (10) and/or the vaccinating agent (21) has a viscosity that is less than 10000 mPa·s, preferably less than 1000 mPa·s, in particular less than 500 mPa·s; and/or higher than 5 mPa·s, preferably higher than 10 mPa·s, in particular higher than 20 mPa·s, measured with a Brookfield viscometer according to EN ISO 2555 at 5 °C.

5. Kit according to one of the preceding claims, **characterized in that** the vaccinating agent (21) has a characteristic color different from both starting materials (7, 10); and/or **in that** the vaccinating agent (21) has a color or no color in contrast to at least one of the starting materials (7, 10).

6. Kit according to one of the preceding claims, **characterized in that** at least one of the starting materials (7, 10) and/or the vaccinating agent (21) comprises a preferably polymeric adjuvant, in particular carbomer, and/or
**in that** a preferably polymeric adjuvant, in particular carbomer, is formed in the vaccinating agent (21); and/or
**in that** the mycoplasma vaccine and/or the circovirus vaccine comprises formaldehyde at a concentration lower than 2.5 mg/m³, preferably lower than 1.5 mg/m³, in particular 0.74 mg/m³ or less.

7. Kit according to one of the preceding claims, **characterized in that** the first and/or second starting material (7, 10) comprises water in a concentration of at least 20 % by weight, preferably at least 30 % by weight, in particular at least 40 % by weight; and/or at most 80 % by weight, preferably at most 70 % by weight, in particular at most 60 % by weight; and/or
**in that** the first starting material (7) and/or second starting material (10) has one or more excipients, in particular preservatives, antioxidants and/or emulsifiers.

8. Kit according to one of the preceding claims, **characterized in that** the first container (1) is filled with the first starting material (7) by an amount of less than 70 %, preferably less than 50 %, in particular less than 45 %; and/or
that the first container (1) comprises gas (8), preferably protective gas, preferably wherein the volume of the gas (8) corresponds at least to the volume of the second starting material (10) in the second container (9), in particular wherein the volume of the gas (8) in the first container (1) exceeds the volume of the second starting material (10) by more than 2 %, preferably more than 5 %, in particular more than 8 % and/or less than 80 %, preferably less than 50 %, in particular less than 40 % or 30 %.

9. Kit according to claim 8, **characterized in that** the adapter device (17) is configured so that during the transport of the second starting material (10) into the first container (1) the gas (8) or protective gas passes from the first container (1) into the second container (9), in particular is transported; and/or
**in that** the second starting material (10) enters the first container (1) under the effect of gravity.

10. Kit according to one of the preceding claims, **characterized in that** the adapter device (17) is configured for fluidic connection of the inner spaces formed by the containers (1, 9), wherein the adapter device (17) has a fluid channel (19) for providing the fluid connection between the inner spaces formed by the containers (1, 9), preferably wherein the adapter device (17) has a channel for venting and/or exhaust which is provided in addition to the fluid channel (19) of the adapter device (17), in particular parallel thereto.

11. Kit according to claim 10, **characterized in that** an opening of the channel for venting and/or exhaust is offset with respect to the outlet opening of the fluid channel (19) on the side facing the first container (1), in the direction of the main extension of the adapter device (17), and/or
**in that** an opening, facing the second container (9), of the channel for venting and/or exhaust is arranged on the side of the outlet opening, facing the second container (9), of the fluid channel (19) which faces the open end, facing the second container (9), of the adapter device (17).

12. Kit according to one of the preceding claims, **characterized in that** the adapter device (17) comprises two adapter elements (18) forming a fluid channel (19) and/or a venting and/or exhaust device, associated with the containers (1, 9), preferably designed as a transfer needle, hollow needle and/or mandrel; and/or
**in that** the adapter device (17) is designed to be specific, preferably to be mechanically specific, to the first container (1) and/or second container (9), preferably wherein the introduction of substances other than the first and/or second starting material (7, 10) into the first and/or second container (1, 9) is prevented; and/or
**in that** the adapter device (17) is designed to provide a connection solely between the first and second containers (1, 9), in particular to the exclusion of the surrounding atmosphere.

13. Kit according to one of the preceding claims, **characterized in that** for the dosage form the first container (1), the second container (9) and the adapter device (17) form a sealed assembly, preferably with a common packaging.

14. Use of a kit according to one of the preceding claims, for the preparation and/or provision of a vaccinating agent (21) for simultaneous immunization against the diseases Porcine Circovirus Disease "PCVD" and Enzootic Pneumonia "EP".

15. Method for providing a vaccinating agent (21) from a first starting material (7) and with a second starting material (10), different from the first, for simultaneous immunization against the diseases Porcine Circovirus Disease "PCVD" and Enzootic Pneumonia "EP" with a kit according to one of claims 1 to 13, wherein a fluid connection is formed between the container (1), which is only partially filled with the first starting material (7), and a second container (9) by the adapter device (17) such that, by means of the adapter device (17), a fluid connection is produced between the first and second containers (1, 9) by breaking through at least one factory-provided closure device (14) of at least one of the containers (1, 9) for a first and/or only time, wherein the second starting material (10) is conveyed through the adapter device (17) and the closure device (5) into the first container (1), and wherein the vaccinating agent (21) is formed in the first container (1) from the first and second starting material (7, 10).

## Revendications

1. Kit pour la préparation d'un produit de vaccination (21) destiné à être utilisé dans l'immunisation contre les maladies Porcine Circovirus Disease "PCVD" et pneumonie enzootique "EP", le kit comprenant un premier produit de départ (7), un deuxième produit de départ (10) différent du premier produit de départ, un premier récipient (1) seulement partiellement rempli avec le premier produit de départ (7), un deuxième récipient (9) comprenant le deuxième produit de départ (10) et un dispositif adaptateur (17) pour établir une liaison de fluide entre le premier et le deuxième récipient (1, 9),
les récipients (1, 9) étant chacun conçus comme une bouteille,
au moins un des récipients (1, 9) étant fermé en usine avec un dispositif de fermeture (5) et le deuxième récipient (9) pouvant être relié au premier récipient (1) au moyen du dispositif adaptateur (17) en rompant le dispositif de fermeture (5) pour la première fois et/ou une seule fois, de telle sorte que le deuxième produit de départ (10) atteint le premier récipient (1) et y forme le produit de vaccination (21) avec le premier produit de départ (7),
les deux produits de départ (7, 10) étant liquides à 2 °C à 8 °C et/ou ayant un point de fusion inférieur à 1°C, le premier produit de départ (7) comprenant seulement un premier des composants vaccin contre les mycoplasmes et vaccin contre le circovirus, le deuxième produit de départ (10) comprenant seulement l'autre des composants vaccin contre les mycoplasmes et vaccin contre le circovirus, et, pour une préparation homogène du produit de vaccination (21) dans le premier récipient (1), le volume total du premier récipient (1) dépasse le volume du premier produit de départ (7) d'au moins le volume du deuxième produit de départ (10).

2. Kit selon la revendication 1, **caractérisé en ce que** le premier produit de départ (7), le deuxième produit de départ (10) et/ou le produit de vaccination (21) est une suspension, de préférence avec des vaccins et/ou des protéines insolubles, de préférence en quantités supérieures à 30 % en poids, de préférence supérieures à 40 % en poids, en particulier supérieures à 50 % en poids ; et/ou inférieures à 90 % en poids, de préférence inférieures à 80 % en poids, en particulier inférieures à 70 % en poids.

3. Kit selon l'une des revendications précédentes, **caractérisé en ce que** le rapport de volume du premier produit (7) au deuxième produit (10) est de 3:1 à 1:3, de préférence de 2:1 à 1:2, en particulier d'environ 1:1.

4. Kit selon l'une des revendications précédentes, **caractérisé en ce que** le premier produit de départ (7) et/ou le deuxième produit de départ (10) et/ou le produit de vaccination (21) présente une viscosité qui est inférieure à 10000 mPa·s, de préférence inférieure à 1000 mPa·s, en particulier inférieure à 500 mPa·s; et/ou supérieure à 5 mPa·s, de préférence supérieure à 10 mPa·s, en particulier supérieure à 20 mPa·s, mesurée avec un viscosimètre Brookfield selon EN ISO 2555 à 5 °C.

5. Kit selon l'une des revendications précédentes, **caractérisé en ce que** le produit de vaccination (21) a une couleur caractéristique différente des deux produits de départ (7, 10) ; et/ou **en ce que** le produit de vaccination (21) a une couleur ou aucune couleur contrairement à au moins un des produits de départ (7, 10).

6. Kit selon l'une des revendications précédentes, **caractérisé en ce qu'**au moins un des produits de départ (7, 10) et/ou le produit de vaccination (21) comprend un adjuvant de préférence polymère, en particulier un carbomère, et/ou
**en ce qu'**un adjuvant de préférence polymère, en particulier un carbomère, est formé dans le produit de vaccination (21) ; et/ou
**en ce que** le vaccin contre les mycoplasmes et/ou le vaccin contre les circovirus contient du formaldéhyde à une concentration inférieure à 2,5 mg/m³, de préférence inférieure à 1,5 mg/m³, en particulier 0,74 mg/m³ ou moins.

7. Kit selon l'une des revendications précédentes, **caractérisé en ce que** le premier et/ou le deuxième produit de départ (7, 10) comprend de l'eau à une concentration d'au moins 20 % en poids, de préférence d'au moins 30 % en poids, en particulier d'au moins 40 % en poids ; et/ou au maximum 80 % en poids, de préférence au maximum 70 % en poids, en particulier au maximum 60 % en poids ; et/ou
**en ce que** le premier produit de départ (7) et/ou le deuxième produit de départ (10) contient une ou plusieurs substances auxiliaires, en particulier des conservateurs, des antioxydants et/ou un émulsifiant.

8. Kit selon l'une des revendications précédentes, **caractérisé en ce que** le premier récipient (1) est rempli avec le premier produit de départ (7) à moins de 70 %, de préférence à moins de 50 %, en particulier à moins de 45 % ; et/ou **en ce que** le premier récipient (1) contient un gaz (8), de préférence un gaz protecteur, le volume du gaz (8) correspondant de préférence au moins au volume du deuxième produit de départ (10) dans le deuxième récipient (9), en particulier le volume du gaz (8) dans le premier récipient (1) dépassant le volume du deuxième produit de départ (10) de plus de 2 %, de préférence de plus de 5 %, en particulier de plus de 8 % et/ou de moins de 80 %, de préférence de moins de 50 %, en particulier de moins de 40 % ou 30 %.

9. Kit selon la revendication 8, **caractérisé en ce que** le dispositif adaptateur (17) est conçu de telle sorte que, lors du transport du deuxième produit de départ (10) dans le premier récipient (1), le gaz (8) ou le gaz de protection passe du premier récipient (1) dans le deuxième récipient (9), en particulier est transporté ; et/ou **en ce que** le deuxième produit de départ (10) entre dans le premier récipient (1) sous l'effet de la gravité.

10. Kit selon l'une des revendications précédentes, **caractérisé en ce que** le dispositif adaptateur (17) est conçu pour la liaison fluidique des espaces intérieurs formés par les récipients (1, 9), le dispositif adaptateur (17) présentant un canal de fluide (19) pour établir la liaison fluidique entre les espaces intérieurs formés par les récipients (1, 9), de préférence le dispositif adaptateur (17) présentant un canal pour l'aération et/ou la ventilation qui est prévu en plus du canal de fluide (19) du dispositif adaptateur (17), en particulier parallèlement à celui-ci.

11. Kit selon la revendication 10, **caractérisé en ce qu'**une ouverture du canal pour l'aération et/ou la ventilation est en retrait par rapport à l'ouverture de sortie du canal de fluide (19) sur le côté faisant face au premier récipient (1) dans la direction de l'extension principale du dispositif adaptateur (17), et/ou
**en ce qu'**une ouverture, faisant face au deuxième récipient (9), du canal pour l'aération et/ou la ventilation est disposée sur le côté de l'ouverture de sortie, faisant face au deuxième récipient (9), du canal de fluide (19) qui fait face à l'extrémité ouverte, faisant face au deuxième récipient (9), du dispositif d'adaptation (17).

12. Kit selon l'une des revendications précédentes, **caractérisé en ce que** le dispositif adaptateur (17) comprend deux éléments adaptateurs (18) formant un canal de fluide (19) et/ou un dispositif d'aération et/ou de ventilation, associés aux récipients (1, 9), de préférence conçus comme une aiguille de transfert, une aiguille creuse et/ou un mandrin ; et/ou
**en ce que** le dispositif adaptateur (17) est conçu spécifiquement, de préférence spécifiquement mécaniquement, pour le premier récipient (1) et/ou le deuxième récipient (9), de préférence en empêchant l'introduction de substances autres que le premier et/ou le deuxième produit de départ (7, 10) dans le premier et/ou le deuxième récipient (1, 9) ; et/ou
**en ce que** le dispositif adaptateur (17) est conçu pour établir une connexion uniquement entre le premier et le deuxième récipient (1, 9), en particulier à l'exclusion de l'atmosphère ambiante.

13. Kit selon l'une des revendications précédentes, **caractérisé en ce que** pour la forme de dosage, le premier récipient (1), le deuxième récipient (9) et le dispositif adaptateur (17) forment un ensemble fermé, de préférence avec un emballage commun.

14. Utilisation d'un kit selon l'une des revendications précédentes, pour la préparation et/ou la fourniture d'un agent vaccinant (21) pour l'immunisation simultanée contre les maladies Porcine Circovirus Disease "PCVD" et pneumonie enzootique "EP".

15. Procédé pour fournir un agent vaccinant (21) à partir d'un premier produit de départ (7) et avec un second produit de départ (10), différent du premier produit de départ, pour l'immunisation simultanée contre les maladies Porcine Circovirus Disease "PCVD" et pneumonie enzootique "EP" avec un kit selon l'une des revendications 1 à 13, une liaison fluidique étant établie entre le récipient (1), qui est seulement partiellement rempli avec le premier produit de départ (7), et un deuxième récipient (9) par le dispositif adaptateur (17) de telle sorte qu'au moyen du dispositif adaptateur (17), une liaison fluidique est établie entre le premier et le deuxième récipient (1, 9) en rompant au moins un dispositif de fermeture (14) prévu en usine d'au moins un des récipients (1, 9) pour une première fois et/ou une seule fois, le deuxième produit de départ (10) étant transporté dans le premier récipient (1) à travers le dispositif adaptateur (17) et le dispositif de fermeture (5), et le produit de vaccination (21) étant formé dans le premier récipient (1) à partir du premier et du deuxième produit de départ (7, 10).
